# EUROPEAN PATENT SPECIFICATION

(11) **EP 2 776 590 B1**
(45) Date of publication and mention of the grant of the patent: **26.10.2016**
(21) Application number: 12784565.9
(22) Date of filing: 06.11.2012
(51) Int. Cl.: C12Q 1/70, G01N 33/576

(54) **PROGNOSTIC METHOD FOR CHECKING EFFICACY OF MICRO RNA-122 INHIBITORS IN HCV+ PATIENTS**
PROGNOSTISCHES VERFAHREN ZUR BEURTEILUNG DER ARZNEIMITTELWIRKSAMKEIT VON MICRO RNA-122 INHIBITOREN IN HCV+ PATIENTEN
PROCÉDÉ PROGNOSTIQUE D'ÉVALUATION DE L'EFFICACITÉ D'INHIBITEURS MICRO RNA-122 EN HCV+ PATIENTS

(30) Priority: 07.11.2011 US 201161556313 P
(43) Date of publication of application: 17.09.2014
(73) Proprietor: Roche Innovation Center Copenhagen A/S, 2970 Hørsholm (DK)
(72) Inventor: HAGEDORN, Peter, 2970 Hørsholm (DK); PETRI, Andreas, 2200 Copenhagen (DK); LINDOW, Morten, 2450 Copenhagen SV (DK); KAUPPINEN, Sakari, 2765 Smoerum (DK)
(74) Representative: Turner, Mark Frederic Paris
(86) International application number: PCT/EP2012/071933
(87) International publication number: WO 2013/068347

(56) References cited:
- DATABASE BIOSIS [Online] BIOSCIENCES INFORMATION SERVICE, PHILADELPHIA, PA, US; October 2011 (2011-10), SU TUNG-HUNG ET AL: "SERUM MICRORNA-122 LEVEL CORRELATES WITH VIROLOGIC RESPONSES TO COMBINATION THERAPY IN CHRONIC HEPATITIS C PATIENTS", XP002691325, Database accession no. PREV201100687270 & HEPATOLOGY, vol. 54, no. Suppl. 1, October 2011 (2011-10), pages 562A-563A, 62ND ANNUAL MEETING OF THE AMERICAN-ASSOCIATION-FOR-THE-STUDY-OF-LIVE R-DISEASES (AASLD); SAN FRANCISCO, CA, USA; NOVEMBER 04 -08, 2011 ISSN: 0270-9139(print)
- LANFORD ROBERT E ET AL: "Therapeutic silencing of microRNA-122 in primates with chronic hepatitis C virus infection", SCIENCE, AMERICAN ASSOCIATION FOR THE ADVANCEMENT OF SCIENCE, WASHINGTON, DC; US, vol. 327, no. 5962, 8 January 2010 (2010-01-08), pages 198-201, XP002595740, ISSN: 0036-8075, DOI: 10.1126/SCIENCE.1178178
- MAGDALENA FILIPOWICZ: "Prognostic potential of hepatic miR-122 measurements and antisense strategies targeting miR-122 as a therapeutic approach in viral hepatitis", LIVER INTERNATIONAL, vol. 31, no. 4, 15 February 2011 (2011-02-15), pages 437-439, XP055051724, ISSN: 1478-3223, DOI: 10.1111/j.1478-3231.2011.02476.x
- LANDGRAF P ET AL: "A Mammalian microRNA Expression Atlas Based on Small RNA Library Sequencing", CELL, CELL PRESS, US, vol. 129, no. 7, 29 June 2007 (2007-06-29) , pages 1401-1414, XP002490508, ISSN: 0092-8674, DOI: 10.1016/J.CELL.2007.04.040
- JOPLING CATHERINE L ET AL: "Modulation of hepatitis C virus RNA abundance by a liver-specific microRNA", SCIENCE, AMERICAN ASSOCIATION FOR THE ADVANCEMENT OF SCIENCE, WASHINGTON, DC; US, vol. 309, no. 5740, 1 September 2005 (2005-09-01), pages 1577-1581, XP009109384, ISSN: 0036-8075, DOI: 10.1126/SCIENCE.1113329
- Verena Bihrer ET AL: "Serum miR-122 as a Biomarker of Necroinfl ammation in Patients With Chronic Hepatitis C Virus Infection", American Journal of Gastroenterology, 24 May 2011 (2011-05-24), pages 1663-1669, XP55051681, Retrieved from the Internet: URL:http://www.nature.com/ajg/journal/v106 /n9/pdf/ajg2011161a.pdf [retrieved on 2013-01-30]

### SEQUENCE LISTING

<110> Santaris Pharma A/S
<120> TARGETED TREATMENT OF HCV AND PROGNOSTIC METHODS
<130> 1133WO
<150> US 61/556313
   <151> 2011-11-07
<160> 3
<170> PatentIn version 3.5
<210> 1
   <211> 85
   <212> RNA
   <213> homo sapiens
<400> 1
   ccuuagcaga gcuguggagu gugacaaugg uguuuguguc uaaacuauca aacgccauua 60
   ucacacuaaa uagcuacugc uaggc 85
<210> 2
   <211> 23
   <212> RNA
   <213> homo sapiens
<400> 2
   uggaguguga caaugguguu ugu 23
<210> 3
   <211> 15
   <212> DNA
   <213> artificial
<220>
   <223> Miravirsen LNA oligonucleotide
<220>
   <221> misc_feature
   <222> (1)..(15)
   <223> phosphorothioate linkages
<220>
   <221> misc_feature
   <222> (1)..(1)
   <223> 5-methyl cytosine LNA
<220>
   <221> misc_feature
   <222> (3)..(3)
   <223> adenosine LNA
<220>
   <221> misc_feature
   <222> (6)..(6)
   <223> guanine LNA
<220>
   <221> misc_feature
   <222> (7)..(7)
   <223> thymine LNA
<220>
   <221> misc_feature
   <222> (10)..(10)
   <223> 5-methyl cytosine LNA
<220>
   <221> misc_feature
   <222> (12)..(12)
   <223> 5-methyl cytosine LNA
<220>
   <221> misc_feature
   <222> (14)..(15)
   <223> 5-methyl cytosine LNA
<400> 3
   ccattgtcac actcc 15

## Description

### FIELD OF INVENTION

The invention relates to the field of diagnostic and prognostic methods for liver function, such as diagnostic and prognostic methods for use in selecting appropriate treatment of hepatitis C. Also disclosed are methods of treatment of subjects infected with HCV which have been identified as likely responders to HCV therapy with a miR-122 inhibitor. The invention also provides for prognostic methods for determining the suitability of treatment of hepatitis C infection with an anti-HCV agent such as a miR-122 inhibitor.

### BACKGROUND

Hepatitis C virus (HCV) is the most common infectious cause of chronic liver disease in Europe and the United States. Worldwide, approximately 3% of the population is estimated to be infected; this corresponds to approximately 200 million people at risk of developing serious liver related morbidity.

microRNA-122 (miR-122) is a liver specific microRNA. miR-122 which is involved in lipid and cholesterol metabolism, and inhibition of miR-122 *in vivo* in rodents results in a reduction in serum cholesterol levels. The molecular mechanisms whereby miR-122 regulated cholesterol metabolism are apparently, at present unknown.

Liver miR-122 level has been inversely correlated to clinical parameters associated with chronic HCV infection (Marquez et al., 2010 Lab Invest December 2010). Blood serum miR-122 has been positively correlated to NAFLD and HCV infection and has been suggested as a biomarker for NAFLD (e.g. Cermelli et al 2011 PLoS ONE August 2011). According to Cermelli et al., there is a lack of correlation between circulating and hepatic miR-122 levels. Serum miR-122 is a biomarker of necroinflammation in HCV infected patients and moderately correlates with serum ALT levels (Bihrer et al Am J Gastroenterol Sept. 2011). Su et al., "Serum MicroRNA-122 Level Correlates with Virologic Responses to Combination Therapy in Chronic Hepatitis C Patients" AASLD November 2011 reports that miR-122 serum levels are correlated to responsiveness to interferon/ribavirin treatment, with high levels of miR-122 serum levels being indicative to responsiveness to treatment.

Miravirsen is a first in class microRNA therapeutic which has shown efficacy for the treatment of hepatitis C (HCV) in both chimpanzees and human patients. Miravirsen is a microRNA-122 inhibitor. The present inventors identified that the sub-population of HCV patients that did not respond to HCV therapy were identified by discriminative blood biomarker assays for liver function.

### SUMMARY OF INVENTION

The surprising observation that a sub-population of HCV patients who do not respond to HCV therapy can be identified by discriminative blood biomarker assays for liver function allows for a prognostic method for screening of HCV infected patients to identify their suitability for HCV therapy.

As disclosed herein, the inhibitor of microRNA-122 (miR-122) may be an antisense oligonucleotide complementary, such as fully complementary, to miR-122 across the length of the oligonucleotide, such as miravirsen.

In some embodiments, the subject is asymptomatic for HCV infection.

In some embodiments, the subject has a chronic hepatitis C infection, wherein the stage of HCV associated fibrosis (or necroinflamation) is less than stage 3 in the Ishak scoring system, or stage F2 in the Metavir stage. Suitably, in some embodiments, the subject does not have incomplete cirrhosis or cirrhosis of the liver, or in some embodiments, bridging fibrosis or portal fibrosis.

The invention provides for a prognostic method for determining the suitability of treatment of a human subject with (e.g.chronic) HCV infection with an inhibitor of microRNA-122, said method comprising the steps of
i) obtained from the human subject
ii) determining the level of at least one biomarker obtained from the human subject in the blood sample
iii) comparing the level of the at least one biomarker with one or more reference samples or reference values;
to determine whether the subject is likely to be or is suitable for treatment of the (e.g. chronic) HCV infection with an inhibitor of microRNA -122. (i.e. a miR-122 inhibitor responder), wherein the at least one biomarker is microRNA-122.

Alternatively put, the above prognostic method may to used to determine whether the subject is likely to respond to (or be responsive to) treatment of the HCV infection with an inhibitor of microRNA -122, such as miravirsen.

The method may be used therefore to identify individual subjects with (e.g. chronic) HCV infection which are likely to be effectively treated (*i.e.* respond to) by administration of an effective dose of the inhibitor of microRNA-122, such as miravirsen.

The prognostic method may be used for identifying non-responders (or accordingly partial responders) to treatment with the inhibitor of microRNA-122.

The above method may also be employed in a method of treatment of (e.g. chronic) HCV, said method comprising the above method and the subsequent step of administering said inhibitor of miR-122 to said subject.

The invention provides for a prognostic kit for in vitro use in the prognostic methods referred to herein, said kit comprising miR-122 detection assay (such as one or more miR-122 detection probes (e.g. one or more miR-122 specific oligonucleotide probe or primers), a quantitative real-time reverse-transcription polymerase chain reaction assay specific for human miR-122 or a detection probe for human miR-122 and at least one further detection assay for at least one further biomarker (as disclosed herein), such as detection assays for GGT, ALT, AST or combinations thereof.

The invention provides for a method of determining the likely effective dose of a miR-122 inhibitory agent (the inhibitor of microRNA-122), such as miravirsen, for administration to a subject with (e.g. chronic) HCV infection, said method comprising
ii) determining the level of at least one biomarker in the blood sample
iii) comparing the level of at least one biomarker with one or more reference samples or reference values,
to determine the likely effective dose of the inhibitor of microRNA-122 for administration to the subject in order to alleviate the (e.g. chronic) HCV infection, wherein the at least one biomarker is microRNA-122.

The invention provides for a detection probe for a microRNA, such as a liver specific microRNA e.g. miR-122, or an assay comprising said detection probe, for use as a companion diagnostic (prognostic) for a HCV therapeutic, such as a microRNA-122 inhibitor (e.g. miravirsen). The companion diagnostic is suitably to be used in a prognostic assay for determining the suitability of a subject in need to treatment of HCV (such as chronic HCV), such as those as described herein.

Further aspects of the invention which are disclosed herein.

### BRIEF DESCRIPTION OF FIGURES

**Figure 1****.** *Distribution of response grade* as a *function of the number of weeks completed in the study.* Horizontal thick lines indicate median. The box includes first to third quartiles. The whiskers indicate the maximum and minimum.
**Figure 2****.** *Distribution of patients at each dose level for each miravirsen response grade.* Shown as a **A.** barplot, and **B,** contingency table.
**Figure 3****.** *Significant associations between observables and miravirsen response grades.* In each subfigure is shown a barplot of average values in each group with error bars indication +/- 1 standard deviation, and a scatterplot of all values at each grade. **A.** miR-122, **B.** GGT, **C.** ALAT, and **D.** ASAT.
**Figure 4****.** *Hierarchical clustering of clinical observables using complete linkage.* Correlation was calculated as Spearman's rank correlation coefficient, r. Distances were calculated as 1-*r*².
**Figure 5****.** *Binary two-decision tree that labels patients* as *either miravirsen responders on non-responders.* The cutoff values for each of the four observables in the tree are presented as values relative to upper limit of normal.
**Figure 6****.** *Data from miravirsen -treated CHC patients support for the binary decision tree.* Scatterplots of CHC patients by **A.** ALAT vs ASAT, and **B.** GGT vs miR-122. Cutoff values from Fig.5 are indicated as dashed lines.

### DETAILED DESCRIPTION OF INVENTION

The invention provides for a method for treatment of HCV, said method comprising administering an effective amount (i.e. a therapeutic dose) of an inhibitor of miR-122, such as miravirsen, to a subject infected with HCV, wherein the subject, prior to treatment, has a serum ALT level below the upper limit of normal.

In some embodiments the subject, has a serum AST level below the upper limit of normal.

In some embodiments the subject, has a serum GGT level below the upper limit of normal.

In some embodiments the subject, has a serum microRNA-122 level of below the upper limit of normal.

In some embodiments the subject, has a serum AST/ALT ratio of 1 or less than 1.

In some embodiments the subject, has a PT value of less than 14 seconds.

The subject may be as defined as the responder referred to herein.

### Normal liver function determination

Liver function may be determined as being normal if, for example, the serum biomarkers are considered below the upper limit of normal. With reference to blood serum biomarkers, such as miR-122, ALT, AST and GGT, normal refers to the central portion (95%) of the normal distribution (*i.e.* within the "normal range"). The upper limit cut off for normal (95%) level is referred to as the "upper limit of normal." It should be recognized that the precise upper limit of normal can vary, e.g. depending upon the reference normal population, gender, age, and in some cases, the specific assay conditions used.

Within the context of the present invention, it should therefore be recognised that "normal liver function" may be determined by the comparison to reference samples or values from a population of subjects with normal liver function. A subject whose blood serum biomarker levels are determined to be above the upper limit of normal may in some embodiments be more likely to be a non-responder or a poor responder.

Alternatively, determination of normal liver function may be determined by selection of suitable pre-determined cut of values for one or more of the biomarker levels, as described herein. The assays used to determine the specific cut off values are identified herein as the Advia Chemistry ALT, AST and GGT protocols, although similar protocols may also be used (taking into account inter-protocol/assay sensitivity/variability). Non-limiting examples of such cut off values are provided herein. As such, the normal range may be considered as being below the levels which are associated with healthy liver function, or cut off values provided herein.

In some embodiments the subject, may have an miR-122, ALT, AST, and/or GGT blood serum level which are below the upper limit of normal.

Within the context of the present invention, it should therefore be recognised that normal liver function" may be determined by the comparison to reference samples or values from one or more subjects (preferably a suitably sized population) with normal liver function. In this regard, determination of normal liver function may be determined by selection of suitable pre-determined cut-off values for one or more of the biomarker levels, as described herein. Non-limiting examples of such cut-off values are provided herein. As such, the normal range may be considered as being below the levels which are associated with non-responders as exemplified and detailed herein. Therefore, in some embodiments, prior to treatment, has an ALAT, ASAT, GammaGT, microRNA-122 level, which is within the normal range. In some embodiments the subject, prior to treatment, has an ALT serum level of less than 69IU/L, and a serum microRNA-122 level of < 3 as determined by deltaCₜ, and optionally a serum GGT level of less than 158U/L.

Alternatively or in combination, normal liver function may be indicated, for example, by the presence of serum biomarkers within a normal range around the mean, for example, 95% of a standard distribution of the mean level of serum biomarker within a healthy population group . The serum biomarkers used to establish a normal liver function in a reference population are preferably, as disclosed herein, for example, miR-122, ALT, AST, GGT and/or AST/ALT ratio. The healthy population group is not infected with HCV, and usually should be demographically similar as, for example normal liver biomarkers may differ between healthy male and female demographic groups.

In the analysis of the clinical data, and as illustrated in the examples:
- Subjects who have pre-treatment ALT levels of below the upper limit of normal are more likely to be responders to treatment with a miR-122 inhibitor such as miravirsen. In some embodiments responder and non-responders or partial responders, may be discriminated by their serum ALT levels, with a ALT level of below the upper limit of normal being indicative of a (likely) responder, and a ALT level of above the upper limit of normal, such as below 1.5x upper limit of normal, such as below 2x upper limit of normal, such as below 3x upper limit of normal, such as below 4x upper limit of normal, such as below 5x upper limit of normal being indicative of a (likely) non-responder or (likely) partial responder.
- Subjects who have pre-treatment AST levels of below the upper limit of normal are more likely to be responders to treatment with a miR-122 inhibitor such as miravirsen. In some embodiments responder and non-responders or partial responders, may be discriminated by their serum AST levels, with a AST level of below the upper limit of normal being indicative of a (likely) responder, and a AST level of above the upper limit of normal, such as below 1.5x upper limit of normal, such as below 2x upper limit of normal, such as below 3x upper limit of normal, such as below 4x upper limit of normal, such as below 5x upper limit of normal being indicative of a (likely) non-responder or (likely) partial responder.
- Subjects who have pre-treatment GGT levels of below the upper limit of normal are more likely to be responders to treatment with a miR-122 inhibitor such as miravirsen. In some embodiments responder and non-responders or partial responders, may be discriminated by their serum GGT levels, with a GGT level of below the upper limit of normal being indicative or a responder, and a GGT level of above the upper limit of normal, such as below 1.5x upper limit of normal, such as below 2x upper limit of normal, such as below 3x upper limit of normal, such as below 4x upper limit of normal, such as below 5x upper limit of normal being indicative of a non-responder or partial responder.
- Subjects who have pre-treatment miR-122 levels of below the upper limit of normal are more likely to be responders to treatment with a miR-122 inhibitor such as miravirsen. In some embodiments responder and non-responders or partial responders, may be discriminated by their serum miR-122 levels, with a serum miR-122 level of below the upper limit of normal being indicative or a (likely) responder, and a miR-122 level of above the upper limit of normal, such as below 1.5x upper limit of normal, such as below 2x upper limit of normal, such as below 3x upper limit of normal, such as below 4x upper limit of normal, such as below 5x upper limit of normal being indicative of a (likely) non-responder or (likely) partial responder.

As is illustrated herein, by combining the assay results for multiple, such as two, three of four serum biomarkers, the accuracy of prediction of responders vs. non-responders or partial responders can be greatly enhanced, and as such the prognostic value may be improved. The following table illustrates some examples of combinations of serum biomarkers which may be used:

| **1 marker** | **2 markers** | **3 markers** | **4 markers** |
|---|---|---|---|
| | | | ALT, AST & GGT & miR-122 |
| | | ALT & AST & miR-122 | |
| | ALT & miR-122 | ALT & GGT & miR-122 | |
| miR-122 | | AST & GGT & miR-122 | |
| | AST & miR-122 | | |
| | GGT & miR-122 | | |

As is illustrated below when combinations of serum bioarkers are used, different classifier structures may be used to optimise the predicitive (prognostic) value.

### Classifiers/Decision Trees

In some embodiments when the levels of the serum biomarkers, such as ALT, AST, miR-122, and/or GGT are detected in a serum sample (from a subject) they may be used to classify that patient as either a responder or non-responder to treatment, via use of a classifier (or decision tree). Such classifiers may be based on a "significant difference" between patterns of ALT, AST, miR-122, and GGT levels in responders and non-responders. Such a significant difference between patterns refers, in different embodiments, to a statistically significant difference, or in other embodiments to a significant difference as recognized by a skilled artisan. Advantageously, the methods of the invention may employ the use of learning and pattern recognition analyzers, clustering algorithms and the like, in order to discriminate between patterns of samples obtained from patients having a condition associated with either non-response or response to treatment. The difference may also be evaluating the pattern of the test sample to a predetermined classification rule or threshold obtained in such a manner. In one embodiment, the classification rule may be in the form of a decision tree, as described in example 5.

### Prognostic Method

The present invention provides for a method for determining the suitability of treatment of a human subject infected with hepatitis C virus (HCV) with an inhibitor of microRNA-122 (e.g. miravirsen). The invention is based upon the surprising observation that liver function markers present in the serum of subjects infected with HCV may be used to discriminate between the sub-population of subjects who respond to said HCV therapy, and those which do not respond to HCV therapy (or respond only partially to HCV therapy). In addition to discriminating between responders and non-responders, the methods of the present invention may be used to determine the [likely] effective dose of said HCV therapy in a human subject infected with HCV (and in need of effective treatment for said HCV infection).

### Serum Biomarkers

Numerous serum biomarkers of liver function are known, by way of a non-limited example the following have been implicated as markers or likely markers of liver function: Lectin-reactive alpha fetoprotein (AFP-L3), Des-gamma-carboxy-prothrombin (DCP), ER6Q, Vimentin, actin alpha 1 skeletal muscle protein, hMFAP 4, tropomyosin, PTGES 2, amyloid P component, transgelin, calponin 1, homo sapiens p20 protein, 17 kDa myosin light chain, H chain H Igg B12, prolyl 4-hydroxylase, beta subunit methylenetetrahydrofolate dehydrogenase 1, PRO2619, aldehyde dehydrogenase 1, fibrinogen alpha chain preproprotein, fructose-bisphosphate aldolase B, argininosuccinate synthetase, Eefla2, AT P 5 Al, alpha-2 actin, regucalcin, serum albumin, mitochondrial malate dehydrogenase, mitochondrial acetoacetyl-CoA thiolase, Hyaluronic Acid (HA), Hepascore, Prothrombin, Gamma Glutamyl Transpeptidase, Apolipoprotein A1 (PGA) index, Age platelet (AP) index, Bonacini index, Pohl score, Forns index, Aspartate aminotransferase/Platelets Ratio index (APRI), MP3 (MMP1, PIINP) index, FIB4,and FibroIndex.

The (blood) serum biomarkers of liver function include for example, microRNA-122 levels (such as SEQ ID NO 2), gamma glutamyltransferase (GammaGT), prothrombin time (PT), alanine aminotransferase (ALAT), and aspartate aminotransferase (ASAT). Other liver function markers are provided herein and the results illustrate that ALT, AST, GGT and microRNA-122 serum levels are the biomarkers of most prognostic value, where as the majority of biomarkers assessed are of little or no predictive value. The liver function markers may, in some embodiments, be associated with liver damage. Whilst not wishing to be limited to any particular theory, the present invention is based upon the observation that HCV-infected subjects who were either non-responsive to miravirsen treatment of were not responsive at a low miravirsen dose (e.g. 3mg/kg/bodyweight or below), had highly elevated biomarkers which are associated with liver damage in their blood, in particular elevated levels of microRNA-122, ALT, AST and/or GGT.
The levels of serum biomarkers are typically assessed prior to treatment. The biomarkers may be serum biomarkers which are indicative of or otherwise associated with liver function. Typically serum biomarkers are evaluated in serum samples taken from the subject. Blood biomarkers may be biomarkers which are present, for example in serum or plasma.

### Time-points for assessment of serum biomarkers

Suitably the level of serum biomarker(s) are assessed prior to treatment with an inhibitor of microRNA-122, e.g. with miravirsen, such as within about 6 months, such as within about 5 months, such as within about 4 months, such as within about 3 months, such as within about 2 months, such as within about 1 month, such as within about 4 weeks, such as within about 3 weeks, such as within about 2 weeks, such as within about 1 week, such as within about 1, 2, 3, 4, 5 or 6 days, prior to the first (or in some embodiments subsequent) administration with the microRNA-122 inhibitor. In some embodiments, the assessment of the level of serum biomarkers involves the step of determining the level of at least one biomarker in the blood sample.
It will be recognised that if, during treatment with the inhibitor of microRNA-122, a subject becomes less responsive to treatment, as measured by an increase in viral titer, the methods of the invention may be employed to determine whether an enhanced dose of the inhibitor of microRNA-122 may be required to restore the effectiveness of the treatment. As such, the methods of the invention may be used to ensure an effective dose of the inhibitor of microRNA-122 is administered to the subject either prior to or during a treatment period.

### Prognostic value of serum microRNAs

In some aspect, the present invention is based upon the surprising finding that serum levels of microRNA-122, are discriminatory for responsiveness to miravirsen treatment. The present invention therefore provides for a method for determining the suitability of treatment of a subject suffering from a disease associated with the expression of, or over-expression of a microRNA, said method comprising the steps of
i) determining the level of at least one microRNA biomarker in the serum sample obtained from a human subject
ii) comparing the level of the at least one microRNA biomarker with one or more reference samples or reference values,
to determine whether the subject is likely to be, or is suitable for, treatment of the disease.

### miR-122 as a serum biomarker

In the present invention it has been discovered that elevated or high levels of serum microRNAs, such as miR-122, in general correlate with decreased miravirsen response grade. This is opposite to what has been reported for treatment with interferon, where decreased levels of liver miR-122 correlate with poor response. Serum miR-122 is therefore a biomarker for responsiveness for treatment with a miR-122 inhibitor, such as miravirsen.

microRNA-122 (miR-122) is a microRNA which is highly abundant in the liver (Lagos-Quintana et al (2002) Current Biol vol 12 pp 735-739) and is a distinct host cellular factor which is reported to be essential for HCV RNA abundance in hepatocytes by forming an oligomeric complex with the 5' non-coding region of HCV (Jopling et al 2005, Machlin et al., PNAS 2011). miR-122 exists in numerous forms, including the mature microRNA (SEQ ID NO 2) and precursors thereto, such as the sequence shown in SEQ ID NO 1. The mature microRNA is known to exist in two forms, one 22nt microRNA sequence shown in SEQ ID NO 2, and a further form where there is an additional 3' U residue.

The miR-122 seed sequence refers to nucleosides 2 through 8 from the
5'-end of the mature miR-122 sequence, *i.e.* 5'-GGAGUGU-3'

According to miRBase, the sequence of the human miR-122 sequence, hsa miR-122, is as follows:
>hsa-mir-122 precursor sequence (miRBase) MI0000442:

The mature hsa-miR-122 sequence (miRBase) MIMAT0000421:
UGGAGUGUGACAAUGGUGUUUG(U) (SEQ ID NO 2)

Serum microRNA levels, such as the levels of mature miR-122 may, as a non-limiting example, can be quantified by extracting RNA from serum samples using but not limited to the methods established by Asuragen's Pharmacogenomics Services Group (Asuragen, Austin, Texas, USA) and quantification of serum miR-122 levels in such RNA samples by RT-qPCR such as TaqMan® MicroRNA Assays (Applied Biosystems; Foster City, CA,) specific.
for miR-122. It is envisaged that other assays may also be applicable, including potential immunological based assays, such as ELISA.

The level of miR-122 present in the serum may be determined as an absolute value or as a ratio to a standard. MicroRNAs biomarkers which may be used as a standard for normalization of miR-122 values include microRNAs whose levels in serum are invariable, such as miR-17, miR-18a, miR-345 and/or combination of miR-17/18a (stable normalization references).

### Exemplary miR 122 cut-off value and ranges:

In some embodiments a deltaCt(miR-122) > 3, such as >4, such as >5, such as >6 such as >7, such as >8 is indicative of a putative non-responder or low-responder.

Delta Ct is defined as threshold cycle (Ct) for mir-122 subtracted from the mean of the threshold cycles of a reference microRNA, such as miR-17 or mir-18a. The Threshold Cycle (Ct) reflects the cycle number at which the fluorescence generated within a qPCR reaction crosses the threshold. The Ct value assigned to a particular well thus reflects the point during the reaction at which a sufficient number of amplicons have accumulated, in that well, to be at a statistically significant point above the baseline.

In some embodiments the level of miR-122 in the blood in a putative non-responder or low responder is greater than 5x upper limit of normal, such as greater than 6x upper limit of normal, such as greater than 7x upper limit of normal, such as greater than 8x upper limit of normal, such as greater than 9x upper limit of normal, such as greater then 9x upper limit of normal, such as greater than 10x upper limit of normal, such as greater than 11 x upper limit of normal, such as greater 12x upper limit of normal, such as greater than 13x upper limit of normal, such as greater than 14x upper limit of normal, such as greater than 15x upper limit of normal the normal blood serum level of miR-122.

MicroRNAs may be detected in a number of ways e.g. by microarrays, northern blots, dot blots, RNAse protection assays, quantitative mass spectroscopy, sequencing, or various quantitative PCR-based techniques. As such, serum microRNA, such as microRNA-122levels may be routinely quantified, as a non-limiting example, by extracting RNA from human serum samples using, but not limited to, the methods established by Asuragen's Pharmacogenomics Services Group (Asuragen, Austin, Texas, USA) followed by quantification of serum miR-122 levels in such RNA samples by RT-qPCR such as TaqMan® MicroRNA Assays (Applied Biosystems; Foster City, CA,) specific for miR-122. Serum microRNA biomarkers, which may be used as standard for normalization of miR-122 values include microRNAs whose expression in serum are invariable, such as miR-17, miR-18a, miR-345 and/or combination of miR-17/18a (stable normalization references).

### Gamma glutamyltransferase (also referred to as GGT herein)

Gamma-glutamyltransferase or gamma-glutamyl transpeptidase (also γ-glutamyltransferase, GGT, GGTP, gamma-GT) (EC 2.3.2.2) is an enzyme that transfers gamma-glutamyl functional groups. It is found in many tissues, the most notable one being the liver, and has significance in medicine as a diagnostic marker. Increased serum gamma-glutamyl transferase (GGT) levels are frequently observed in chronic hepatitis C virus (HCV) infection, and elevated GGT is an indirect marker of more advanced liver disease in chronic hepatitis C.
Blood test results for GGT suggest that the population 97.5th percentile (the so-called "upper limit of normal") is about 45 IU/L for women and about 75 IU/L for men, although will depend on the reference population and the assay used.

### Exemplary GammaGT cut-off value/ranges:

In some embodiments the level of GGT in the serum of a putative non/partial responder is >1.5x upper limit of normal, such as > 2x upper limit of normal, such as >3x upper limit of normal, such as >4x upper limit of normal, such as >5x upper limit of normal.

In some embodiments, the level of GGT for a female putative non/partial responder is > 60 IU/L, such as >70 IU/L, such as >80 IU/L, such as >90 IU/L such as > 100 IU/L.

In some embodiments, the level of GGT for a male putative non/partial responder is > 100 IU/L, such as >110 IU/L, such as >120 IU/L, such as >130 IU/L such as >140 IU/L.

### Prothrombin time

The prothrombin time (PT) is a measure of the extrinsic pathway of blood coagulation. It is used to determine the clotting tendency of blood, and can be used to determine the extent of liver damage (liver function) as well as other factors. Using a standard assay, the reference range for prothrombin time is usually around 10-13 seconds. An elevated PT, for example 17 seconds, is indicative of serious liver damage. In some embodiments the PT level of a putative non/partial responder is greater than >1x normal such as >1.1x normal, such as >1.2x normal, such as >1.3x normal, such as >1.4x normal, such as >1.5x normal.

### Alanine aminotransferase (also referred to as ALT or ALAT herein)

*Background:* alanine amino transferase (ALAT), also known as Alanine Transaminase (ALT) or serum glutamic pyruvic transaminase (sGPT), is a homodimeric cytoplasmic pyridoxal phosphate-dependent enzyme involved in cellular nitrogen metabolism, amino acid metabolism, and liver gluconeogenesis. ALT mediates conversion of major intermediate metabolites, catalyzing reversible transamination between alanine and α-ketoglutarate to form pyruvate and glutamate. ALT is widely distributed in many tissues but is found in greatest abundance in the liver. The major role of ALT in the liver is the conversion of alanine to glucose which is then exported to the body to be utilized in a multitude of processes.
*ALT Measurement:* Measurement of ALT activity is generally carried out by monitoring the rate of NADH oxidation in a coupled reaction system employing lactate dehydrogenase (LDH). The oxidation of NADH to NAD+ is accompanied by a decrease in absorbance at 340 nm. Under circumstances in which the ALT activity is rate limiting, the rate decrease is directly proportional to the ALT activity in the sample. A protocol for measuring ALT may be the Advia Chemistry Systems ALT assay (03815151 Rev. B 2007-05).

ALT is commonly measured clinically as a part of a diagnostic evaluation of hepatocellular injury, to determine liver function. When used in diagnostics, it is almost always measured in international units/liter (U/L). While sources vary on specific normal range values, most show between 5-60 U/L as being normal. When elevated ALT levels are found in the blood, the possible underlying causes can be further narrowed down by measuring other enzymes. For example, elevated ALT levels due to liver-cell damage can be distinguished from biliary duct problems by measuring alkaline phosphatase. Elevated ALT is often associated with advancement of HCV related diseases and liver damage, and prior to the introduction of immunology-based tests, elevated ALT was used to screen for potential HCV infection.

In some embodiments the level of ALT in the blood serum of a putative non/partial responder is(e.g. when using the Advia Chemistry Systems assay) >69 IU/L, such as >70 IU/L, such as >75 IU/L, such as >80 IU/L, such as >90 IU/L, such as >100 IU/L, such as >110 IU/L, such as >120 IU/L, such as >130 IU/L, such as >150 IU/L, such as >200 IU/L.

In some embodiments the level of ALT in the serum of a putative non/partial responder is >1.3x normal, such as >1.4x normal, such as >1.5x normal, such as > 2x normal, such as >3x normal, such as >4x normal, such as >5x normal.

### Aspartate aminotransferase (Also referred to as ASAT herein)

*Background:* Aspartate aminotransferase (ASAT), also known as Aspartate Transaminase (AST) or serum glutamic oxaloacetic transaminase (SGOT), is a pyridoxal phosphate (PLP)-dependent transaminase enzyme. AST catalyzes the reversible transfer of an a-amino group between aspartate and glutamate and, as such, is an important enzyme in amino acid metabolism. AST is found in the liver, heart, skeletal muscle, kidneys, brain, and red blood cells, and it is commonly measured clinically as a marker for liver health.
*Measurement:* In general, AST activity assay is based on the quantification of oxaloacetate produced by AST. In this assay, oxaloacetate and NADH are converted to malate and NAD by the enzyme malate dehydrogenase. The decrease in NADH absorbance at 340 nm is proportionate to AST activity.
*Basic methodology:* See for example, Bergmeyer H.U., Scheibe P. and Wahlefeld A.W. (1978). Optimization of methods for aspartate aminotransferase and alanine aminotransferase. Clin. Chem. 24(1): 58-73 or Bowers Jr G.N. and McComb R.B. (1984). A unifying reference system for clinical enzymology: aspartate aminotransferase and the International Clinical Enzyme Scale. Clin. Chem. 30(7): 1128-1136. A protocol for measuring ALT may be the Advia Chemistry Systems ALT assay (03903166 Rev. B 2007-05).
In some embodiments the level of AST in the serum of a putative non/partial responder is >1.3x normal, such as >1.4x normal, such as >1.5x normal, such as > 2x normal, such as >3x normal, such as >4x normal, such as >5x normal.

In some embodiments the level of AST in the blood (serum/plasma) indicative of non or partial responders (e.g. when using the Advia Chemistry Systems assay) >50 IU/L, such as >55IU/L, such as >60IU/L, such as >65IU/L, such as >70IU/L, such as >75IU/L, such as >80IU/L, such as >85IU/L, such as >90IU/L, such as >95IU/L, such as > 100IU/L.

### AST/ALT ratio:

It has been reported that an AST:ALT > or = 1 is highly specific but not diagnostic for the presence of cirrhosis in patients with chronic HCV infection. The ratio reflects the grade of fibrosis in these patients. In some embodiments the AST/ALT ratio in the blood serum of a putative non/partial responder is at least 1, such as >1, such as >1.1, such as >1.2, such as >1.3, such as >1.4, such as >1.5.

### Detection probes and assays for microRNAs:

The invention provides for a detection probe (or pair of detection probes) for one or more microRNAs, such as one or more liver-specific microRNAs, such as (including) microRNA-122 (e.g. mature miR-122), for use determining the likely suitability or suitability of a subject for treatment with a HCV therapeutic, such as the microRNA-122 inhibitor, for example miravirsen. As is exemplified with microRNA-122 inhibitor herein, the level of liver specific microRNA in serum may be inversely correlated with the suitability of the subject for treatment.

Therefore, the detection probe of the invention may be used as a companion diagnostic. The detection probes may be in the form of a kit, also referred to as a prognostic kit herein. The detection probe(s) may therefore form part of a quantification assay. A kit may further comprise quantification assays, e.g. for other serum biomarkers referred to herein, e.g. ALT, AST and or GGT, and/or one or more reference microRNAs, e.g. one or more microRNA whose levels are invariable in the serum.

MicroRNAs, such as microRNA-122 may be detected in a sample using hybridisation based techniques. A microRNA-122 detection probe may, for example, be an oligonucleotide (oligomer) which comprises a contiguous nucleobase sequence which complementary to at least 6, such as 7, 8, 9, 10, 11, 12, 13, 14, 15, 16, 17, 18, 19, 20, 21, 22 or 23 contiguous nucleotides present in the mature microRNA-122 sequence. The oligomer may be as defined herein with regards the microRNA-122 inhibitor-for example may be modified by the use of nucleoside analogues, such as LNA. The detection probe may be the same length as contiguous nucleobase sequence or in some embodiments longer, for example, up to 30, such as up to 40, such as up to 50 nucleotides in length.

Detection probes typically comprises a recognition sequence complementary to a nucleotide target, such as an RNA (or DNA) target sequence. Detection probes may be labelled - i.e. comprise one or more labels. The term "label" as used herein refers to any atom or molecule which can be used to provide a detectable (preferably quantifiable) signal, and which can be attached to a nucleic acid or protein. Labels may provide signals detectable by fluorescence, radioactivity, colorimetric, X-ray diffraction or absorption, magnetism, enzymatic activity, immune-reactivity, and the like. The detection element of the detection probes according to the invention may be single or double labelled (e.g. by comprising a label at each end of the probe, or an internal position). In one aspect, the detection probe comprises two labels capable of interacting with each other to produce a signal or to modify a signal, such that a signal or a change in a signal may be detected when the probe hybridizes to a target sequence. A particular aspect is when the two labels comprise a quencher and a reporter molecule. A particular detection aspect of the invention referred to as a "molecular beacon with a stem region" is when the recognition segment is flanked by first and second complementary hairpin-forming sequences which may anneal to form a hairpin. A reporter label is attached to the end of one complementary sequence and a quenching moiety is attached to the end of the other complementary sequence. The stem formed when the first and second complementary sequences are hybridized (i.e., when the probe recognition segment is not hybridized to its target) keeps these two labels in close proximity to each other, causing a signal produced by the reporter to be quenched by fluorescence resonance energy transfer (FRET). The proximity of the two labels is reduced when the probe is hybridized to a target sequence and the change in proximity produces a change in the interaction between the labels. Hybridization of the probe thus results in a signal (e.g. fluorescence) being produced by the reporter molecule, which can be detected and/or quantified. In the present context, the term "label" means a reporter group, which is detectable either by itself or as a part of a detection series. Examples of functional parts of reporter groups are biotin, digoxigenin, fluorescent groups (groups which are able to absorb electromagnetic radiation, e.g. light or X-rays, of a certain wavelength, and which subsequently reemits the energy absorbed as radiation of longer wavelength; illustrative examples are DANSYL (5-dimethylamino)-1-naphthalenesulfonyl), DOXYL (N-oxyl-4,4-dimethyloxazolidine), PROXYL (N-oxyl-2,2,5,5-tetramethylpyrrolidine), TEMPO (N-oxyl-2,2,6,6-tetramethylpiperidine), dinitrophenyl, acridines, coumarins, Cy3 and Cy5 (trademarks for Biological Detection Systems, Inc.), erythrosine, coumaric acid, umbelliferone, Texas red, rhodamine, tetramethyl rhodamine, Rox, 7-nitrobenzo-2-oxa-1-diazole (NBD), pyrene, fluorescein, Europium, Ruthenium, Samarium, and other rare earth metals), radio isotopic labels, chemiluminescence labels (labels that are detectable via the emission of light during a chemical reaction), spin labels (a free radical (e.g. substituted organic nitroxides) or other paramagnetic probes (e.g. Cu 2+, Mg 2+) bound to a biological molecule being detectable by the use of electron spin resonance spectroscopy). Especially interesting examples are biotin, fluorescein, Texas Red, rhodamine, dinitrophenyl, digoxigenin, Ruthenium, Europium, Cy5, Cy3, etc.

MicroRNA Expression Detection Methods (Wang, Zhiguo, Yang, Baofeng) Springer ISBN 978-3-642-04927-9 provides a review of microRNA detection methods which may be used to detect and quantify microRNAs in a sample, for example miR-122. Methods include miRNA arrays, northern blots, RT-PCR, miR-Q RT-PCR, stem-loop RT-PCR.
Examples of hybridisation/PCR based assays include:
- miRCURY LNA™ microRNA (miRNA) Detection Probes/arrays (Exiqon A/S)
- Qiagen miScript PCR system
- Ambion mirVana™ qRT-PCR miRNA Detection Kit,
- AB Taqman miRNA assay
- Mir-X miRNA qRT-PCR SYBR® Kits (Clonetech)

Other assays include
- ELISA approach, e.g. by HFH
- Signosis miRNA Plate Assay (and similar streptavidin/biotin HRP chemilumiescent assays)

When using qPCR (qRT-PCR), one measure of the expression level of a specific microRNA is the "threshold cycle value", denoted Ct, obtained by real-time qRT-PCR, as described in the examples. Another measure of the expression level of a specific microRNA is the "crossing point value", denoted Cp, likewise obtained by real-time qRT-PCR. The Ct and Cp measures of microRNA expression levels provides approximately similar measures, see Bustin SA (editor) A-Z of quantitative PCR, IUL Biotechnology Series 5 (2004). The use of Ct or Cp depends on the machine on which the assay is performed. If the amplification is performed on a LightCycler 480 Real-Time PCR system the expression level of a specific microRNA is by use of Cp. If the amplification is performed on a Applied Biosystems ABI Prism 7900HT instrument the expression of a specific microRNA is by use of Cp.

### Reference Samples or values

In some embodiments, the level of at least one biomarker in the blood sample obtained from the subject with HCV infection is compared to the level of the at least one biomarker with a reference sample or value.
For use in the prognostic method, typically the reference sample or value is obtained from at least one subject (suitable a population of subjects) with a known or pre-determined level of the biomarker and a known responsiveness to said treatment (such as the inhibitor of microRNA-122, e.g. miravirsen). In some embodiments, the level of at least one biomarker is compared to a database of reference values obtained from a population of subjects with a known or pre-determined level of the biomarker and a known responsiveness to said treatment. In some embodiments the reference value may be the value which represents the upper limit of normal, as described herein, or numerical values (multiples) thereof. Therefore, in some embodiments, the reference value may be a pre-determined cut-off value determined from a database of reference values. Examples of suitable cut-off values are provided herein. It should be recognised that as the database becomes more comprehensive the precision by which individual cut-off values may be assigned will improve. Furthermore, as is illustrated herein, the actual cut-off value assigned to any one blood biomarker will depend on the context in which that blood biomarker level is being assayed, for example the selection of other blood biomarkers and the classifier structure used in the comparison step.

In some respects, the reference value is the normal level or range of said at least one biomarker.

In the diagnostic methods described herein, the reference sample or value is obtained from at least one subject (suitable a population of subjects) with a known or pre-determined level of the biomarker and a known stage of progression of hepatitis C in a human subject, such as the level of necroinflammation in the liver (e.g. as assessed by liver biopsy histology as described herein).

In some embodiments the invention relates to a computer system comprising a database of said reference values and a program for comparison of the biomarker value(s) obtained from the blood sample obtained from the subject with the database of reference values. The computer program may, in some embodiments, comprise one or more classifier algorithm.

### Examples of cut-off Values/Classifiers

A putative non/partial responder is a subject whose levels of serum biomarkers are indicative of a likelihood (or tendency) that the subject may be a non or partial responder. As described herein, by combining the serum biomarker values for several, such as 2, 3, 4, 5 or 6 biomarkers, the confidence level achieved in designating non or partial responders can be greatly enhanced. In this regard, whilst biomarkers such as miR-122, ALT, AST, GammaGT and PT are excellent indicators of a putative non-responder, the ability to accurately discriminate between non-responders, partial responders and responders is greatly enhanced by integrating several biomarkers.
In some embodiments the level of at least 2 biomarkers are determined, such as at least 2 biomarkers listed herein, such as selected from the group consisting of, miR-122, GammaGT, ASAT, and ALAT (or ASAT/ALAT ratio).

In some embodiments the level of at least 3 biomarkers are determined, such as at least 3 biomarkers listed herein, such as selected from the group consisting of, blood clotting (e.g. PT), miR-122, GammaGT, ASAT, and ALAT (or ASAT/ALAT ratio).

In some embodiments the level of at least 4 biomarkers are determined, such as at least 4 biomarkers listed herein, such as selected from the group consisting of, blood clotting (e.g. PT), miR-122, GammaGT, ASAT, and ALAT (or ASAT/ALAT ratio).

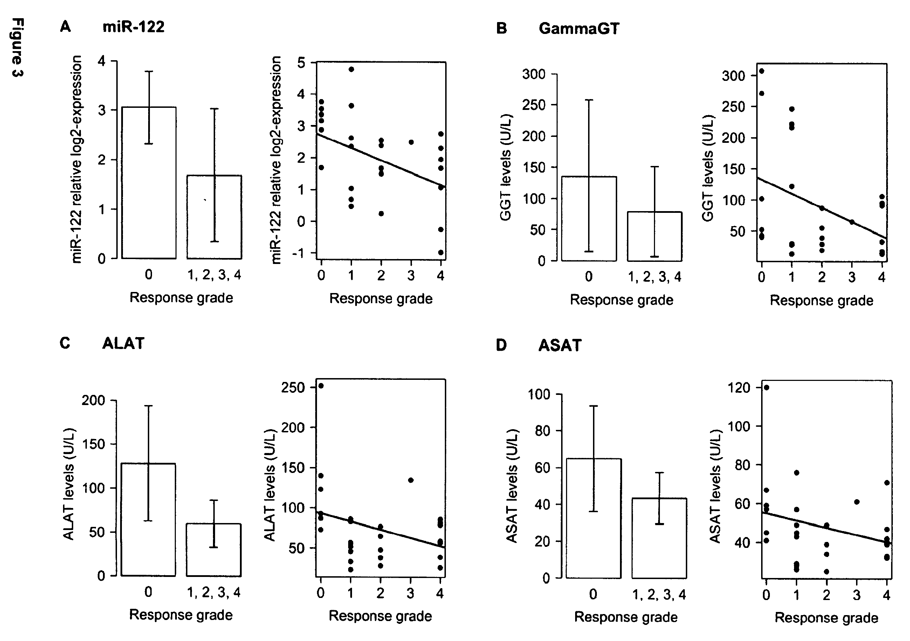

As is exemplified herein the specific combinations of the biomarkers which are discriminatory for non-responders or partial responders, can be grouped into classifiers which integrate the results obtained from the different biomarker assays to enable remarkably accurate predictions of which subjects are responders, partial responders or non-responders. By a non-limiting example, based on the results obtain, one particularly useful classifier is shown in Figure 3, where a normal level of ALAT (e.g. 69 IU/L or less) is predictive of a responder, whereas a level of ALAT >69 IU/L may be predictive of a non-responder, but is fully predictive of a non-responder when there is either an elevated level of miR-122 (such as a -deltaC_{T}of >3), or an elevated GGT (such as >158 IU/L). Thus, as illustrated in the examples, it is possible to with remarkable accuracy predict which subject are non-responders and which are responders to treatment with the inhibitor of microRNA-122.

### HCV Infection/Viral Titer Assays

The presence of HCV RNA in serum or liver is the first evidence of HCV infection. HCV RNA is detectable in serum by PCR within days to eight weeks following exposure, depending in part upon the size of the inoculums.
Molecular virological techniques play a key role in diagnosis and monitoring of treatment. Because it is difficult to culture the virus, hepatitis C virus infection is confirmed by the detection of viral RNA through nucleic acid tests (NATs), and these tests are used to monitor the response to antiviral therapy (Scott JD and Gretch DR. Molecular diagnostics of hepatitis C virus infection. J Am Med Assn. 2007;297:724-32.).
Anti-HCV ELISA tests become positive as early as eight weeks after exposure. Approximately one-half of patients with symptomatic acute infection have detectable antibodies to HCV by ELISA when first presenting. However, the development of HCV antibodies may be delayed in patients who have subclinical infection.
Nucleic acid tests directly detect the presence of HCV RNA using a combination of amplification and detection techniques. Except for certain uncommon clinical situations, NATs have supplanted the recombinant immunoblot assay as the preferred test to confirm HCV infection. Nucleic acid tests are classified into qualitative tests (qualitative polymerase chain reaction [PCR], transcription-mediated amplification [TMA]), and quantitative tests (branched-chain DNA [bDNA]) amplification, quantitative PCR/realtime PCR).

In standard HCV therapy, all candidates for antiviral therapy should be tested for viral titer using HCV RNA quantification assays. Both target amplification based assays, and signal amplification branched assays are used routinely, with ranges of quantification between 10¹ and 10⁶ IU(mL). For documentation of a virologic response at the end of therapy (end-of-treatment response) or an SVR (sustained viral response) 6 months after completing therapy, a more sensitive quantitative HCV RNA assay (such as real-time TaqMan polymerase chain reaction, with a sensitivity threshold of ∼50 IU/mL) or a qualitative HCV RNA assay (based on polymerase chain reaction or transcription-mediated amplification, with lower quantitation limits of 50 ∼IU/mL) is recommended. HCV quantification assays are routinely used to demonstrate virological response to treatment, and typically the same assay is used prior to and subsequent to treatment.
Typically, for interferon based therapies, the level of HCV is a prognostic indicator of the likelihood of response (Dienstag and McHutchinson, AGA Vol 130, pp 231-264). In the present study, it was found that for miravirsen treatment, the level of HCV had less prognostic value as compared to the serum biomarkers referred to herein.

### The Subject

The subject is typically a human being who is infected with HCV. In some embodiments the subject has chronic HCV infection.

Typically the subject is or has been diagnosed with hepatitis C infection. In this regard, the blood biomarkers referred to herein may be performed at the same time as the diagnostic tests for HCV infection, or subsequent to the diagnosis of HCV infection.

The subject may be infected with HCV of a genotype selected from the group consisting of 1 a, 1 b, 2, 3, 4, 5 or 6. In some embodiments the genotype of the HCV is 1a. In some embodiments the genotype of the HCV is 1 b.

The subject may be a human in need of effective treatment for chronic hepatitis C.

Effective treatment is determined by at least a one-log reduction (i.e. 10 fold reduction) in HCV viral titres as described herein (see below under responders/non-responders). In some embodiments, effective treatment is at least two logs reduction (i.e. 100 fold). Partial responders typically illustrate a reduction of between one and two-log reduction.

In some embodiments the subject may be treatment naive, *i.e.* has no history of therapeutic intervention for treatment of HCV infection.

In some embodiments the subject has been recently diagnosed, e.g. within the previous two years, or within the previous year, or within 6 months, such as within 3 months.

In some embodiments, the subject may be asymptomatic or only mildly symptomatic.

In some embodiments the stage of HCV associated fibrosis (or necroinflamation) exhibited by the subject is less than stage 3 in the Ishak scoring system, or stage F2 in the Metavir stage. Suitably, in some embodiments, the subject does not have incomplete cirrhosis or cirrhosis of the liver, or in some embodiments, bridging fibrosis or portal fibrosis.

In some embodiments mildly symptomatic may refer to subjects who have symptoms which correspond to (or subject which have been diagnosed with) less than stage 3 or 4 in the Ishak scale, or less than stage 2 or 3 in the METAVIR scale.

### Non-responders and responders

*Response Grade:* Response to treatment with the inhibitor of microRNA-122, such as miravirsen, in each patient is graded on a scale from 0 to 4. This grade is assigned based on the log-base-10 maximal reduction in HCV compared to baseline.

Specifically:
(i) Grade 0: less than 1 log decrease in HCV viral load,
(ii) Grade1: more than 1 log and less that 2 log decrease in viral load,
(iii) Grade 2: more than 2 log and less than 3 log decrease in viral load,
(iv) Grade 3: more than 3 log and less that 4 log decrease in viral load,
(v) Grade 4: more than 4 log decrease in HCV viral load. A grade between 1 and 2 is considered to be a partial responder. Partial responders may still benefit from HCV therapy, particularly if an elevated dose of the HCV therapeutic, such as the miR-122 inhibitor (e.g. miravirsen) is given.
Serum HCV levels may be determined using Roche Diagnostics Taqman assay (e.g. COBAS® AmpliPrep/COBAS® TaqMan® HCV Test).

### Non-invasive diagnostic assays

Traditionally, the level of necroinflammation in a HCV infected subject is determined by liver biopsy, an invasive and painful procedure which is widely used in the United States to provide information on the state of the liver and prognostic information for future disease progression (Dienstag and McHutchinson, AGA Vol 130, pp 231-264). Interferon-based therapies are traditionally used only when the liver biopsy indicated moderate to severe fibrosis (stage 3 or above in the Ishak scoring system, or stage F2 or above in the Metavir stage)

**Table 2. Histologic Scoring Systems for Fibrosis**

| Fibrosis | METAVIR¹⁰⁵ | Ishak¹⁰⁴ |
|---|---|---|
| None | 0 | 0 |
| Portal fibrosis (some) | 1 | 1 |
| Portal fibrosis (most) | 1 | 2 |
| Bridging fibrosis (occasional) | 2 | 3 |
| Bridging fibrosis (marked) | 3 | 4 |
| Incomplete cirrhosis | 4 | 5 |
| Cirrhosis | 4 | 6 |

If the liver biopsy indicates milder histological disease, progression may be considered sufficiently slow to justify monitoring without imminent interferon treatment. However, as indicated in the present invention, treatment of miravirsen may be particularly suitable for early stage of disease as such subjects are more likely to have serum biomarkers indicative of responders. Percutaneous liver biopsy is associated with potential complications, including bleeding (1%-3%),pain (20%-30%), bile peritonitis (<1%), pneumothorax(<1%), punctured viscera (<1%), and death.

The remarkable prognostic value of the present invention illustrates that for treatment with a microRNA-122 inhibitor, such as miravirsen, a liver biopsy can be avoided. The clinical data reported herein also indicates that successful miravirsen treatment may be associated with milder HCV disease stages, and as such the present invention may also have significant diagnostic value in determining the stage of HCV disease progression, and may therefore be a non-invasive alternative to liver biopsy.

In another aspect, the invention provides a diagnostic method for determining the stage of progression of hepatitis C in a human subject, such as the level of necroinflammation in the liver, said method comprising the steps of
i) obtaining a blood sample from the human subject infected with HCV
ii) determining the level of mircoRNA-122 in the blood sample, and at least one further biomarker in the blood sample
iii) comparing the level of microRNA and the at least one further biomarker with a reference sample or one or more reference values obtained from one or more subjects with a known stage of progression of hepatitis C (such as level of necroinflammation) to determine the stage of progression of hepatitis C in the subject.

Suitably the reference values are in the form of a database of reference values obtained from a population of subjects with known stage of progression of hepatitis C infection.

By way of example, at least one further biomarker is selected from the group consisting of ALT, AST, GGT, ALT and GGT, ALT and AST, AST and GGT, and ALT, AST and GGT.

The stage of progression of HCV may, for example be determined as according to the METAVIR or Ishak scale (Ishak K, et al. Histological grading and staging of chronic hepatitis. J Hepatol 1995; 22:696-699; Bedossa P & Poynard T, French METAVIR Cooperative Study Group. An algorithm for grading activity in chronic hepatitis C. Hepatology 1994;24:289-293).

It is also recognized that the level of necroinflammation may be determined in mammalian subjects which are not infected with HCV, and as such the invention provides for a method for determining the level of liver function in a subject, said method comprising the steps of
i) obtaining a blood sample from a mammalian subject, such as a human
ii) determining the level of mircoRNA-122 in the blood sample, and at least one further biomarker in the blood sample
iii) comparing the level of microRNA and the at least one further biomarker with a reference sample or one or more reference values obtained from one or more subjects with a known level of liver function to determine the level of liver function in said subject.

By way of example, at least one further biomarker is selected from the group consisting of ALT, AST, GGT, ALT and GGT, ALT and AST, AST and GGT, and ALT, AST and GGT. The invention therefore provides for the use of a microRNA-122 quantification assay for determining the degree of liver function in a mammalian subject, wherein the use is in combination with said at least one further biomarker in the blood sample. In some embodiments, the subject may be infected with HCV. In some embodiments, the subject may not be infected with HCV. In some embodiments, the subject may be diagnosed with a disorder selected from the group consisting of hepatitis, such as hepatitis B, C and D, non-alcoholic fatty liver disease and non-alcoholic steatohepatitis, cytomegalovirus infection, schistosomiasis infection and Leptospirosis infection.

### Method for determining the likely effective dose of the inhibitor of microRNA-122, such as Miravirsen

The data obtained from the phase2a clinical trial indicates that some subjects do not respond to miravirsen treatment, particularly at the low dose of miravirsen (e.g. 3 mg/kg). The correlation between responsiveness to miravirsen and dose is indicative of heterogeneity within HCV-infected patient populations for responsiveness to miravirsen treatment, and as such the present invention provides for a method for determining the likely effective dose of a miR-122 inhibitory agent (the inhibitor of microRNA-122), such as miravirsen, for administration to a subject with chronic HCV infection, said method comprising
ii) determining the level of at least one biomarker in a serum sample obtained from a human subject
iii) comparing the level of at least one biomarker with one or more reference samples or reference values,
to determine at the likely effective dose of the inhibitor of microRNA-122 for administration to the subject in order to alleviate the chronic HCV infection wherein the at least one biomarker is microRNA-122.

The above method may be combined with the prognostic method - for example, subjects with one or more blood biomarker scores which are outside of the normal range, but are not above the set cut-off value for designation as a non-responder, may be effectively treated using a higher dose of miravirsen, e.g. at the 5mg/kg or 7mg/kg dose. Alternatively the period of treatment may be extended.

The term "[therapeutically] effective amount" in general refers to an amount required to reduce symptoms of the disease in an individual. The dose will be adjusted to the individual requirements in each particular case. That dosage can vary within wide limits depending upon numerous factors such as the severity of the disease to be treated, the age and general health condition of the patient, other medicaments with which the patient is being treated, the route and form of administration and the preferences and experience of the medical practitioner involved. Generally, in some embodiments, treatment is initiated with smaller dosages which are less than the optimum dose of the compound. Thereafter, the dosage may be increased by small increments until the optimum effect for the individual patient is reached. One of ordinary skill in treating diseases described herein will be able, without undue experimentation and in reliance on personal knowledge, experience and the disclosures of this application, to ascertain a therapeutically effective amount of the compounds of the present invention for a given disease and patient.

A (e.g. daily/weekly/monthly dose) may, for example be, between about 0.1 and about 500 mg/kg body weight, such as between 0.1 and about 100 mg/kg body weight such as between 0.1 and 1 mg/kg body weight per day, or between 1.0 and about 10 mg/kg body weight per day. Thus, for administration to a 70 kg person, in some embodiments, the dosage range may be about 7 mg to 0.7 g per day. In some embodiments each dose of the oligomer, such as miravirsen may, for example, be between about 0.1 mgs/kg or 1 mg/kg and about 10mg/kg of 20mgs/kg, (i.e. a range of between e.g.0.1 and 20mg/kg, such as between 1 mg/kg and 12mg/kg). Individual doses may therefore be, e.g. about 0.5mgs/kg, such as about 0.6mgs/kg, such as about 0.7mgs/kg, such as about 0.8mgs/kg, such as about 0.9mgs/kg, such as about 1 mg/kg, such as about 2mgs/kg, such as about 3mgs/kg, such as about 4mgs/kg, such as about 5mgs/kg, such as about 6mgs/kg, such as about 7mgs/kg, such as about 8mgs/kg, such as about 9mgs/kg, such as about 10mgs/kg. In some embodiments the dose of the oligomer is below 7mg/kg, such as below 5mg/kg or below 3mg/kg. In some embodiments the dose of the oligomer is above 0.5mg/kg, such as above 1 mg/kg.

In some embodiments, the time interval between each administration of the miR-122 inhibitor such as miravirsen during the treatment period may be for example, selected from the group consisting of 1 day, 2 days, 3 days, 4 days, five days, six days and weekly. In some embodiments the time interval between administration is at least every other day, such as at least every three days, such as at least every 4 days, such as at least every 5 days, such as at least every 6 days, such as weekly, such as at least every two weeks (biweekly) or at least every 3 or 4 weeks, or at least monthly.

The oligomer, e.g. miravirsen, may, for example, be administered parentally. For parenteral, subcutaneous, intradermal or transderman administration the formulation may include a sterile diluent, buffers, regulators of tonicity and antibacterials. The oligomer may, for example be administered i.v.i or s.c. in a saline solution. For intravenous or sub cutaneous administration the preferred carriers are physiological saline or phosphate buffered saline. Other methods of administration may be used, for example oral, nasal, rectal administration.

### Compositions

The oligomer may be used in pharmaceutical formulations and compositions. Suitably, such compositions comprise a pharmaceutically acceptable diluent, carrier, salt or adjuvant. PCT/DK2006/000512 provides suitable and preferred pharmaceutically acceptable diluent, carrier and adjuvants. Suitable dosages, formulations, administration routes, compositions, dosage forms, combinations with other therapeutic agents, pro-drug formulations are also provided in WO2007/031091. Miravirsen sodium is a preferred pharmaceutical composition.

In some embodiments, the inhibitor of microRNA-122 is administered in water or saline water. In some embodiments, the inhibitor of microRNA-122 is administered via a parenteral route of administration, such as intravenous or sub-cutaneous. In some embodiments, the administration route is via oral administration (see WO2011/048125 ).

The inhibitor of microRNA-122 may be, in some embodiments, in a unit formulation (*i.e.* unit dose) such as in a pharmaceutically acceptable carrier or diluent in an amount sufficient to deliver to a patient a therapeutically effective amount without causing serious side effects in the treated patient..

The dosage of the pharmaceutical composition is dependent on severity and responsiveness of the disease state to be treated, and the course of treatment lasting from several days to several months, or until a cure is effected or a diminution of the disease state is achieved. Optimal dosing schedules can be calculated from measurements of drug accumulation in the body of the patient. Optimum dosages may vary depending on the relative potency of individual oligonucleotides. Generally it can be estimated based on EC₅₀s found to be effective in *in vitro* and *in vivo* animal models. In general, dosage is from 0.01 µg to 1 g per kg of body weight, and may be given once or more daily, weekly, or monthly. The repetition rates for dosing can be estimated based on measured residence times and concentrations of the drug in bodily fluids or tissues.

### The Anti-HCV Agent

The anti-HCV agent may be an inhibitor of microRNA-122 (a miR-122 inhibitor). Efficacy of a microRNA inhibitor, such as an antisense oligonucleotide, may be determined by measuring the downstream effects of inhibiting microRNA-122 activity in vivo. For example, de-repression of direct microRNA-122 target mRNAs (secondary indicies) or serum cholesterol (tertiary indices) *in vivo,* for example in mice or in primates (Elmén et al. Nature 2008). However, the discovery that the mechanism of miR-122 interaction with the HCV virus is fundamentally distinct from the mechanism of miR-122 mediated mRNA repression provides strong evidence that assays based on *in vivo* inhibition of mRNA targets of miR-122 are likely to be inadequate for prediction of clinically relevant inhibition of HCV replication. In this regard, inhibitors of microRNA-122 which may be effective in treatment of HCV in vivo typically are highly effective in de-repressing mRNA targets (e.g. at least 3 fold de-repression of AldoA or Ndrg3 *in vivo* in mouse), and/or highly effective in lowering serum cholesterol (e.g. by about at least 30 - 40% in mice and non-human primates - see Elmén et al, Nature 2008). Preferred anti-HCV agents include miR-122 inhibitors, such as antisense oligomers: Furthermore, small molecule inhibitors of microRNA-122 have been described previously,

### miR-122 inhibitor - small molecule inhibitors

As reported in Young et al., JACS 2010, 132, 7976-7981) ), it is possible to assay for small molecule inhibitors of miR122 and small molecule inhibitors of miR-122 are known, such as those illustrated below:

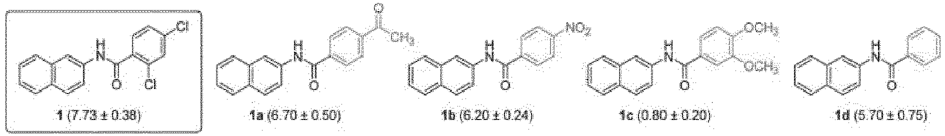

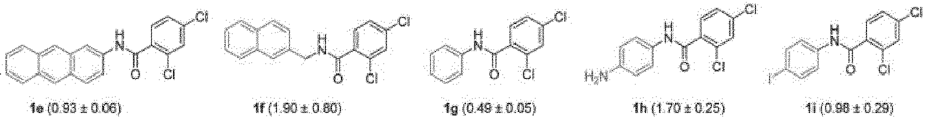

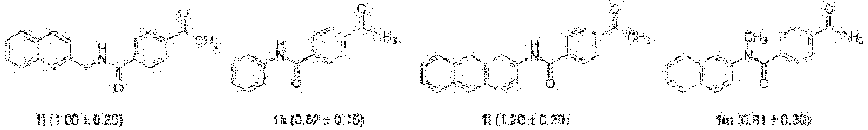

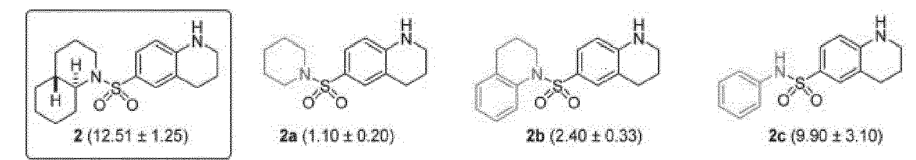

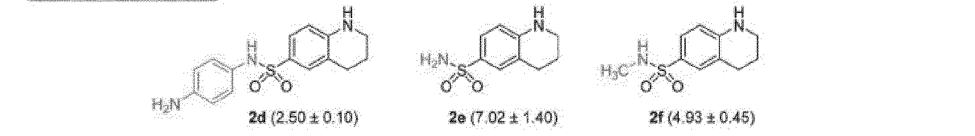

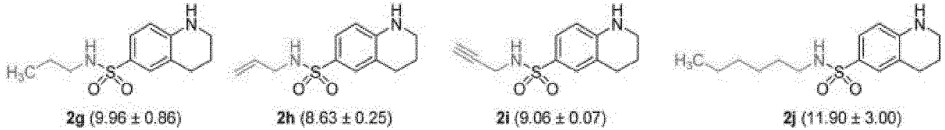

The numerical values refer to luciferase expression due to miR-122 deprepression, and values greater than 1 indicate miR-122 inhibition.

### miR-122 inhibitor - Antisense Oligomers

The miR-122 inhibitor may be an antisense oligomer. In some embodiments, the anti-HCV agent is an antisense oligomer targeting the mature hsa-microRNA-122.

The antisense oligomer may comprise at least 6, such as at least 7 consecutive nucleobases which are complementary to a part of a miR-122 sequence, such as the mature hsa-miR-122 sequence.

In some embodiments, the antimiR-122 oligonucleotide may be designed so that is essentially incapable of recruiting RNAseH, for example by employing a mixmer or totalmer design. Oligonucleotides that are essentially incapable of recruiting RNAseH are well known in the literature, in example see WO2007/112754, WO2007/112753, or WO2009/043353. Mixmers may be designed to comprise a mixture of affinity enhancing nucleotide analogues, such as in non-limiting example 2'-O-alkyl-RNA monomers, 2'-amino-DNA monomers, 2'-fluoro-DNA monomers, LNA monomers, arabino nucleic acid (ANA) mononmers, 2'-fluoro-ANA monomers, HNA monomers, 3 fluoro hexitol monomers (3F HNA), INA monomers, 2'-MOE-RNA (2'-O-methoxyethyl-RNA), 2'Fluoro-DNA, and LNA. In a further embodiment, the oligonucleotide does not include any DNA or RNA nucleotides, but is solely composed of affinity enhancing nucleotide analogues, such a molecule is may also be termed a totalmer. In some embodiments, the mixmer only comprise one type of affinity enhancing nucleotide analogues together with DNA and/or RNA. In some embodiments, the oligonucleotide is composed solely of one or more types of nucleotide analogues, such as in non-limiting example 2'-O-alkyl-RNA monomers, 2'-amino-DNA monomers, 2'-fluoro-DNA monomers, LNA monomers, arabino nucleic acid (ANA) mononmers, 2'-fluoro-ANA monomers, HNA monomers, INA monomers, 2'-MOE-RNA (2'-O-methoxyethyl-RNA), 2'Fluoro-DNA, and LNA.

### Length

In some embodiments the antisense oligonucleotide has a length of 7 - 25 (contiguous) nucleotides, such as 8, 9, 10, 11, 12, 13, 14, 15, 16, 17, 18, 19, 20, 21, 22, 23, or 24 (contiguous) nucleotides. In some embodiments, the antisense oligonucleotide has a length of 7 - 10 (contiguous) nucleotide, or in some instances 7 - 16 nucleotides. In some embodiments, the antisense oligonucleotide at least 8 (contiguous) nucleotides in length, between 10-17 or 10 - 16 or 10-15 (contiguous) nucleotides, such as between 12 - 15 (contiguous) nucleotides.

### Oligomers which are essentially incapable of recruiting RNAseH

EP 1 222 309 provides in vitro methods for determining RNaseH activity, which may be used to determine the ability to recruit RNaseH. A oligomer is deemed capable of recruiting RNase H if, when provided with the complementary RNA target, it has an initial rate, as measured in pmol/l/min, of at least 1 %, such as at least 5%, such as at least 10% or less than 20% of the equivalent DNA only oligonucleotide, with no 2' substitutions, with phosphorothioate linkage groups between all nucleotides in the oligonucleotide, using the methodology provided by Example 91 - 95 of EP 1 222 309.

In some embodiments, an oligomer is deemed essentially incapable of recruiting RNaseH if, when provided with the complementary RNA target, and RNaseH, the RNaseH initial rate, as measured in pmol/l/min, of at least 0.5%, is less than 1%, such as less than 5%,such as less than 10% or less than 20% of the initial rate determined using the equivalent DNA only oligonucleotide, with no 2' substitutions, with phosphorothioate linkage groups between all nucleotides in the oligonucleotide, using the methodology provided by Example 91 - 95 of EP 1 222 309.

It should be recognised that oligonucleotides which are mixmers or totalmers are usually essentially incapable of recruiting RNAseH and as such where we use the term essentially incapable or recruiting RNaseH herein, in some embodiments, such a term may be replaced with the term mixmer or totalmer, as defined herein, even if, in some instances such oligomers actually do possess significant ability to recruit RNaseH, such as when using DNA mixmers with alpha-L-oxy-LNA.

### Totalmers

In some embodiments, the oligomer or contiguous nucleotide sequence thereof consists of a contiguous sequence of nucleotide analogues, such as affinity enhancing nucleotide analogues - referred to herein is as a 'totalmer'.

A totalmer is a single stranded oligomer which only comprises non-naturally occurring nucleotides. The oligomer maybe a totalmer- indeed various totalmer designs are highly effective as therapeutic oligomers, particularly when targeting microRNA (antimiRs) or as splice switching oligomers (SSOs).

In some embodiments, the totalmer comprises or consists of at least one XYX or YXY sequence motif, such as a repeated sequence XYX or YXY, wherein X is LNA and Y is an alternative (i.e. non LNA) nucleotide analogue, such as a 2'-OMe RNA unit and 2'-fluoro DNA unit. The above sequence motif may, in some embodiments, be XXY, XYX, YXY or YYX for example.

In some embodiments, the totalmer may comprise or consist of a contiguous nucleotide sequence of between 8 and 16 nucleotides, such as 9, 10, 11, 12, 13, 14, or 15 nucleotides, such as between 8 and 12 nucleotides.

In some embodiments, the contiguous nucleotide sequence of the totalmer comprises of at least 30%, such as at least 40%, such as at least 50%, such as at least 60%, such as at least 70%, such as at least 80%, such as at least 90%, such as 95%, such as 100% LNA units. The remaining units may be selected from the non-LNA nucleotide analogues referred to herein in, such those selected from the group consisting of 2'-O_alkyl-RNA unit, 2'-OMe-RNA unit, 2'-amino-DNA unit, 2'-fluoro-DNA unit, LNA unit, PNA unit, HNA unit, INA unit, and a 2'MOE RNA unit, or the group 2'-OMe RNA unit and 2'-fluoro DNA unit.

In some embodiments the totalmer consist or comprises of a contiguous nucleotide sequence which consists only of LNA units.

In some embodiments, the totalmer may be targeted against a microRNA (i.e. be antimiRs) - as referred to in US provisional applications 60/979217 and 61/028062, and PCT/DK2008/000344.

### Mixmers

The term 'mixmer' refers to oligomers which comprise both naturally and non-naturally occurring nucleotides, where, as opposed to gapmers, tailmers, headmers and blockmers, there is no contiguous sequence of more than 5 naturally occurring nucleotides, such as DNA units.

The oligomer according to the invention maybe a mixmer - indeed various mixmer designs are highly effective as therapeutic oligomers, particularly when targeting microRNA (antimiRs), microRNA binding sites on mRNAs (Blockmirs) or as splice switching oligomers (SSOs).

The oligomer may, in some embodiments, also be a mixmer and indeed, due to the ability of mixmers to effectively and specifically bind to their target, the use of mixmers as therapeutic oligomers are considered to be particularly effective in decreasing the target RNA.

In some embodiments, the mixmer comprises or consists of a contiguous nucleotide sequence of repeating pattern of nucleotide analogue and naturally occurring nucleotides, or one type of nucleotide analogue and a second type of nucleotide analogues. The repeating pattern, may, for instance be every second or every third nucleotide is a nucleotide analogue, such as LNA, and the remaining nucleotides are naturally occurring nucleotides, such as DNA, or are a 2'substituted nucleotide analogue such as 2'MOE or 2'fluoro analogues as referred to herein, or, in some embodiments selected form the groups of nucleotide analogues referred to herein. It is recognised that the repeating pattern of nucleotide analogues, such as LNA units, may be combined with nucleotide analogues at fixed positions - e.g. at the 5' or 3' termini.

In some embodiments the first nucleotide of the oligomer, counting from the 3' end, is a nucleotide analogue, such as an LNA nucleotide.

In some embodiments, which may be the same or different, the second nucleotide of the oligomer, counting from the 3' end, is a nucleotide analogue, such as an LNA nucleotide.

In some embodiments, which maybe the same or different, the seventh and/or eighth nucleotide of the oligomer, counting from the 3' end, are nucleotide analogues, such as LNA nucleotides.

In some embodiments, which may be the same or different, the ninth and/or the tenth nucleotides of the oligomer, counting from the 3' end, are nucleotide analogues, such as LNA nucleotides.

In some embodiments, which may be the same or different, the 5' terminal of the foligomer is a nucleotide analogue, such as an LNA nucleotide.

The above design features may, in some embodiments be incorporated into the mixmer design, such as antimiR mixmers.

In some embodiments, the mixmer does not comprise a region of more than 4 consecutive DNA nucleotide units or 3 consecutive DNA nucleotide units. In some embodiments, the mixmer does not comprise a region of more than 2 consecutive DNA nucleotide units.

In some embodiments, the mixmer comprises a region consisting of at least two consecutive nucleotide analogue units, such as at least two consecutive LNA units.

In some embodiments, the mixmer comprises a region consisting of at least three consecutive nucleotide analogue units, such as at least three consecutive LNA units.

In some embodiments, the mixmer of the invention does not comprise a region of more than 7 consecutive nucleotide analogue units, such as LNA units. In some embodiments, the mixmer of the invention does not comprise a region of more than 6 consecutive nucleotide analogue units, such as LNA units. In some embodiments, the mixmer of the invention does not comprise a region of more than 5 consecutive nucleotide analogue units, such as LNA units. In some embodiments, the mixmer of the invention does not comprise a region of more than 4 consecutive nucleotide analogue units, such as LNA units. In some embodiments, the mixmer of the invention does not comprise a region of more than 3 consecutive nucleotide analogue units, such as LNA units. In some embodiments, the mixmer of the invention does not comprise a region of more than 2 consecutive nucleotide analogue units, such as LNA units.

In the mixmer embodiments, which refer to the modification of nucleotides in positions 3 to 8, counting from the 3' end, the LNA units may be replaced with other nucleotide anlogues, such as those referred to herein. "X" may, therefore be selected from the group consisting of 2'-O-alkyl-RNA unit, 2'-OMe-RNA unit, 2'-amino-DNA unit, 2'-fluoro-DNA unit (2'fluoro), 2'-MOE-RNA (2'MOE) unit, LNA unit, PNA unit, HNA unit, INA unit. "x" is preferably DNA or RNA, most preferably DNA.

In some embodiments, the mixmer, such as an antimiR mixmer, is modified in positions 3 to 8 - i.e. comprises at least one nucleotide analogue in positions 3 to 8, counting from the 3' end. The design of this sequence may be defined by the number of non-LNA units present or by the number of LNA units present. In some embodiments of the former, at least one, such as one, of the nucleotides in positions three to eight, counting from the 3' end, is a non-LNA unit. In some embodiments, at least two, such as two, of the nucleotides in positions three to eight, counting from the 3' end, are non-LNA units. In some embodiments, at least three, such as three, of the nucleotides in positions three to eight, counting from the 3' end, are non-LNA units. In some embodiments, at least four, such as four, of the nucleotides in positions three to eight, counting from the 3' end, are non-LNA units. In some embodiments, at least five, such as five, of the nucleotides in positions three to eight, counting from the 3' end, are non-LNA units. In some embodiments, all six nucleotides in positions three to eight, counting from the 3' end, are non-LNA units.

The oligomer may, in some embodiments, be either i) fully complementary to a sub-sequence of contiguous nucleotides present in the miRNA target, or ii) comprises no more than a single mismatch with the complement of a sub-sequence of contiguous nucleotides present in said RNA target. As such the oligonucleotide is an antisense oligonucleoitde - in that it is either fully complementary to the corresponding region of the target sequence, or comprises no more than a single mismatch with the corresponding region of the target sequence. The RNA target is typically associated with a medical condition or disease, and may in some embodiments, be a microRNA or a mRNA, for example. The oligomer may therefore be, for example, an antimiR, a microRNA mimic, a microRNA blockmir, or an antisense oligomer.

The oligomer may therefore be an antimir which targets (i.e. comprises or consists of a contiguous nucleotide sequence which is fully complementary to (a corresponding region of) microRNA-122 or comprises of no more than a single mismatch thereto. Such oligonucleotides may be referred to as anti-microRNA oligonucleotides.

### Examples of modulators of microRNA-122 useful in the invention

Specially preferred compounds for use in the present invention are those that target microRNA-122. The sequence of miR-122 can be found in the microRNA database "mirbase" (http://microrna.sanger.ac.uk/sequences/). Inhibitors of microRNA-122 have been described in numerous patents and articles and are well known to the person skilled in the art. In a some embodiments, examples of such documents describing useful microRNA-122 modulators are WO2007/112754, WO2007/112753, or WO2009/043353. In some embodiments, such microRNA-122 modulators are those described in WO2009/20771, WO2008/91703, WO2008/046911, WO2008/074328, WO2007/90073, WO2007/27775, WO2007/27894, WO2007/21896, WO2006/93526, WO2006/112872, WO2005/23986, or WO2005/13901, or WO2010/122538 .

### Miravirsen (SPC3649)

In a preferred embodiment the antisense oligomer is Miravirsen (SPC3649) which has the formula.

5'-**^{m}C_{S}^{o}**C_{S}**A_{S}^{o}**t_{S}t_{S}**G_{S}^{o}T_{S}^{o}**c_{S}a_{S}**^{m}C_{S}^{o}**a_{S}**^{m}C_{S}^{o}t**_{S}**^{m}C_{S}^{om}C^{o}**-3'

wherein; a lowercase letter identifies a DNA unit, and an upper case letter identifies a LNA unit, **^{m}C** identifies a 5-methylcytosine LNA, subscript ₛ identifies a phosphorothioate internucleoside linkage, and wherein LNA units are beta-D-oxy, as identified by a ^{o} superscript after LNA residue.

Miravirsen is the first microRNA-targeted drug to enter clinical trials. Miravirsen may be used as monotherapy or in combination with direct acting antiviral agents as an interferon-free treatment for chronic HCV infection in multiple genotypes.

The term "oligomer" in the context of the present invention, refers to a molecule formed by covalent linkage of two or more nucleotides (*i.e.* an oligonucleotide). Herein, a single nucleotide (unit) may also be referred to as a monomer or unit. In some embodiments, the terms "nucleoside", "nucleotide", "unit" and "monomer" are used interchangeably. It will be recognised that when referring to a sequence of nucleotides or monomers, what is referred to is the sequence of bases, such as A, T, G, C or U.

The oligomer typically consists or comprises of a contiguous nucleotide sequence of from 7 - 25 units.

In various embodiments, the compound of the invention does not comprise RNA (units). It is preferred that the compound according to the invention is a linear molecule or is synthesised as a linear molecule. The oligomer is a single stranded molecule, and preferably does not comprise short regions of, for example, at least 3, 4 or 5 contiguous nucleotides, which are complementary to equivalent regions within the same oligomer (*i*.*e*. duplexes) - in this regards, the oligomer is not (essentially) double stranded. In some embodiments, the oligomer is essentially not double stranded, such as is not a siRNA. In various embodiments, the oligomer of the invention may consist entirely of the contiguous nucleotide region. Thus, the oligomer is not substantially self-complementary.

The terms "corresponding nucleotide analogue" and "corresponding nucleotide" are intended to indicate that the nucleotide in the nucleotide analogue and the naturally occurring nucleotide are identical. For example, when the 2-deoxyribose unit of the nucleotide is linked to an adenine, the "corresponding nucleotide analogue" contains a pentose unit (different from 2-deoxyribose) linked to an adenine.

The terms "reverse complement", "reverse complementary" and "reverse complementarity" as used herein are interchangeable with the terms "complement", "complementary" and "complementarity".

### Nucleosides and Nucleoside analogues

In some embodiments, the terms "nucleoside analogue" and "nucleotide analogue" are used interchangeably.

The term "nucleotide" as used herein, refers to a glycoside comprising a sugar moiety, a base moiety and a covalently linked group (linkage group), such as a phosphate or phosphorothioate internucleotide linkage group, and covers both naturally occurring nucleotides, such as DNA or RNA, and non-naturally occurring nucleotides comprising modified sugar and/or base moieties, which are also referred to as "nucleotide analogues" herein. Herein, a single nucleotide (unit) may also be referred to as a monomer or nucleic acid unit.

In field of biochemistry, the term "nucleoside" is commonly used to refer to a glycoside comprising a sugar moiety and a base moiety, and may therefore be used when referring to the nucleotide units, which are covalently linked by the internucleotide linkages between the nucleotides of the oligomer. In the field of biotechnology, the term "nucleotide" is often used to refer to a nucleic acid monomer or unit, and as such in the context of an oligonucleotide may refer to the base - such as the "nucleotide sequence", typically refer to the nucleobase sequence (*i.e.* the presence of the sugar backbone and internucleoside linkages are implicit). Likewise, particularly in the case of oligonucleotides where one or more of the internucleoside linkage groups are modified, the term "nucleotide" may refer to a "nucleoside" for example the term "nucleotide" may be used, even when specifiying the presence or nature of the linkages between the nucleosides.

As one of ordinary skill in the art would recognise, the 5' terminal nucleotide of an oligonucleotide does not comprise a 5' internucleotide linkage group, although may or may not comprise a 5' terminal group.

Non-naturally occurring nucleotides include nucleotides which have modified sugar moieties, such as bicyclic nucleotides or 2' modified nucleotides, such as 2' substituted nucleotides.

"Nucleotide analogues" are variants of natural nucleotides, such as DNA or RNA nucleotides, by virtue of modifications in the sugar and/or base moieties. Analogues could in principle be merely "silent" or "equivalent" to the natural nucleotides in the context of the oligonucleotide, *i.e.* have no functional effect on the way the oligonucleotide works to inhibit target gene expression. Such "equivalent" analogues may nevertheless be useful if, for example, they are easier or cheaper to manufacture, or are more stable to storage or manufacturing conditions, or represent a tag or label. Preferably, however, the analogues will have a functional effect on the way in which the oligomer works to inhibit expression; for example by producing increased binding affinity to the target and/or increased resistance to intracellular nucleases and/or increased ease of transport into the cell. Specific examples of nucleoside analogues are described by *e.g.* Freier & Altmann; Nucl. Acid Res., 1997, 25, 4429-4443 and Uhlmann; Curr. Opinion in Drug Development, 2000, 3(2), 293-213, and in Scheme 1:

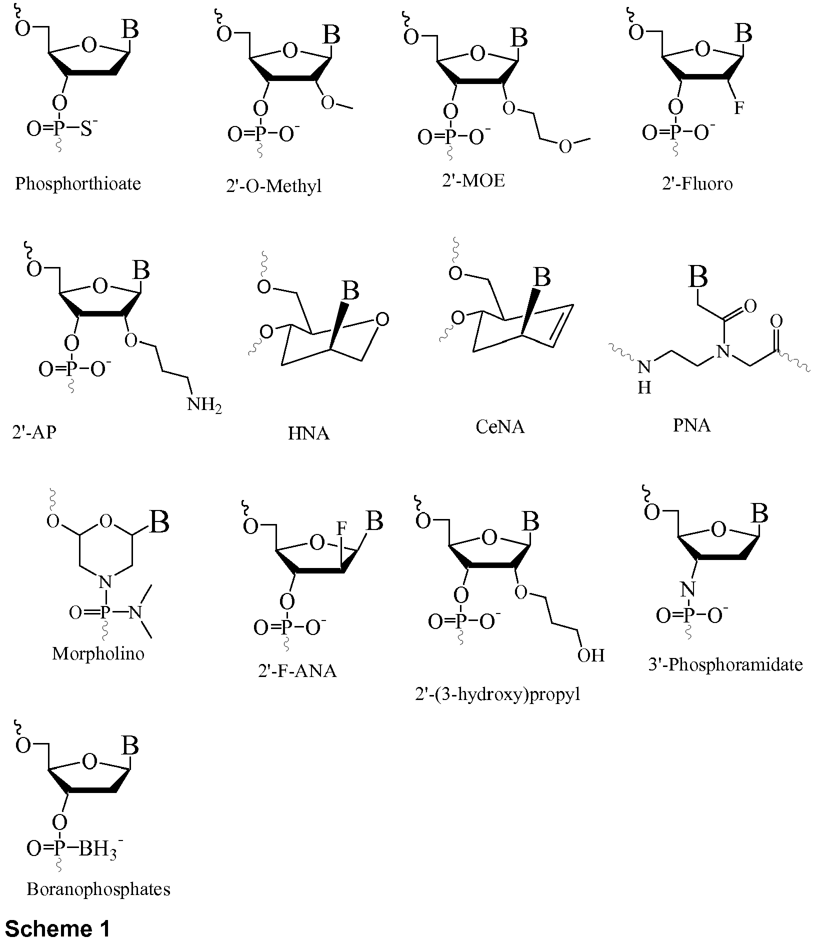

The oligomer may thus comprise or consist of a simple sequence of natural occurring nucleotides - preferably 2'-deoxynucleotides (referred to here generally as "DNA"), but also possibly ribonucleotides (referred to here generally as "RNA"), or a combination of such naturally occurring nucleotides and one or more non-naturally occurring nucleotides, *i.e.* nucleotide analogues. Such nucleotide analogues may suitably enhance the affinity of the oligomer for the target sequence.

Examples of suitable and preferred nucleotide analogues are provided by WO2007/031091 or are referenced therein.

Incorporation of affinity-enhancing nucleotide analogues in the oligomer, such as LNA or 2'-substituted sugars, can allow the size of the specifically binding oligomer to be reduced, and may also reduce the upper limit to the size of the oligomer before non-specific or aberrant binding takes place.

In some embodiments, the oligomer comprises at least 1 nucleoside analogue. In some embodiments the oligomer comprises at least 2 nucleotide analogues. In some embodiments, the oligomer comprises from 3-8 nucleotide analogues, *e.g*. 6 or 7 nucleotide analogues. In the by far most preferred embodiments, at least one of said nucleotide analogues is a locked nucleic acid (LNA); for example at least 3 or at least 4, or at least 5, or at least 6, or at least 7, or 8, of the nucleotide analogues may be LNA. In some embodiments all the nucleotides analogues may be LNA.

It will be recognised that when referring to a preferred nucleotide sequence motif or nucleotide sequence, which consists of only nucleotides, the oligomers of the invention which are defined by that sequence may comprise a corresponding nucleotide analogue in place of one or more of the nucleotides present in said sequence, such as LNA units or other nucleotide analogues, which raise the duplex stability/Tₘ of the oligomer/target duplex (*i.e.* affinity enhancing nucleotide analogues).

In some embodiments, any mismatches between the nucleotide sequence of the oligomer and the target sequence are preferably found in regions outside the affinity enhancing nucleotide analogues, such as region B as referred to herein, and/or region D as referred to herein, and/or at the site of non modified such as DNA nucleotides in the oligonucleotide, and/or in regions which are 5' or 3' to the contiguous nucleotide sequence.

Examples of such modification of the nucleotide include modifying the sugar moiety to provide a 2'-substituent group or to produce a bridged (locked nucleic acid) structure which enhances binding affinity and may also provide increased nuclease resistance.

A preferred nucleotide analogue is LNA, such as oxy-LNA (such as beta-D-oxy-LNA, and alpha-L-oxy-LNA), and/or amino-LNA (such as beta-D-amino-LNA and alpha-L-amino-LNA) and/or thio-LNA (such as beta-D-thio-LNA and alpha-L-thio-LNA) and/or ENA (such as beta-D-ENA and alpha-L-ENA). Most preferred is beta-D-oxy-LNA.

In some embodiments the nucleotide analogues present within the oligomer of the invention (such as in regions A and C mentioned herein) are independently selected from, for example: 2'-O-alkyl-RNA units, 2'-amino-DNA units, 2'-fluoro-DNA units, LNA units, arabino nucleic acid (ANA) units, 2'-fluoro-ANA units, HNA units, INA (intercalating nucleic acid -Christensen, 2002. Nucl. Acids. Res. 2002 30: 4918-4925) units and 2'MOE units. In some embodiments there is only one of the above types of nucleotide analogues present in the oligomer of the invention, or contiguous nucleotide sequence thereof.

In some embodiments the nucleotide analogues are 2'-O-methoxyethyl-RNA (2'MOE), 2'-fluoro-DNA monomers or LNA nucleotide analogues, and as such the oligonucleotide of the invention may comprise nucleotide analogues which are independently selected from these three types of analogue, or may comprise only one type of analogue selected from the three types. In some embodiments at least one of said nucleotide analogues is 2'-MOE-RNA, such as 2, 3, 4, 5, 6, 7, 8, 9 or 10 2'-MOE-RNA nucleotide units. In some embodiments at least one of said nucleotide analogues is 2'-fluoro DNA, such as 2, 3, 4, 5, 6, 7, 8, 9 or 10 2'-fluoro-DNA nucleotide units.

In some embodiments, the oligomer according to the invention comprises at least one Locked Nucleic Acid (LNA) unit, such as 1, 2, 3, 4, 5, 6, 7, or 8 LNA units, such as from 3 - 7 or 4 to 8 LNA units, or 3, 4, 5, 6 or 7 LNA units. In some embodiments, all the nucleotide analogues are LNA. In some embodiments, the oligomer may comprise both beta-D-oxy-LNA, and one or more of the following LNA units: thio-LNA, amino-LNA, oxy-LNA, and/or ENA in either the beta-D or alpha-L configurations or combinations thereof. In some embodiments all LNA cytosine units are 5'methyl-Cytosine. In some embodiments of the invention, the oligomer may comprise both LNA and DNA units. Preferably the combined total of LNA and DNA units is 10-25, such as 10 - 24, preferably 10-20, such as 10 - 18, even more preferably 12-16. In some embodiments of the invention, the nucleotide sequence of the oligomer, such as the contiguous nucleotide sequence consists of at least one LNA and the remaining nucleotide units are DNA units. In some embodiments the oligomer comprises only LNA nucleotide analogues and naturally occurring nucleotides (such as RNA or DNA, most preferably DNA nucleotides), optionally with modified internucleotide linkages such as phosphorothioate.

The term "nucleobase" refers to the base moiety of a nucleotide and covers both naturally occuring a well as non-naturally occurring variants. Thus, "nucleobase" covers not only the known purine and pyrimidine heterocycles but also heterocyclic analogues and tautomeres thereof.

Examples of nucleobases include, but are not limited to adenine, guanine, cytosine, thymidine, uracil, xanthine, hypoxanthine, 5-methylcytosine, isocytosine, pseudoisocytosine, 5-bromouracil, 5-propynyluracil, 6-aminopurine, 2-aminopurine, inosine, diaminopurine, and 2-chloro-6-aminopurine.

In some embodiments, at least one of the nucleobases present in the oligomer is a modified nucleobase selected from the group consisting of 5-methylcytosine, isocytosine, pseudoisocytosine, 5-bromouracil, 5-propynyluracil, 6-aminopurine, 2-aminopurine, inosine, diaminopurine, and 2-chloro-6-aminopurine.

### LNA

The term "LNA" refers to a bicyclic nucleoside analogue, known as "Locked Nucleic Acid". It may refer to an LNA monomer, or, when used in the context of an "LNA oligonucleotide", LNA refers to an oligonucleotide containing one or more such bicyclic nucleotide analogues. LNA nucleotides are characterised by the presence of a linker group (such as a bridge) between C2' and C4' of the ribose sugar ring - for example as shown as the biradical R^{4*} - R^{2*} as described below.

The LNA used in the oligonucleotide compounds of the invention preferably has the structure of the general formula I 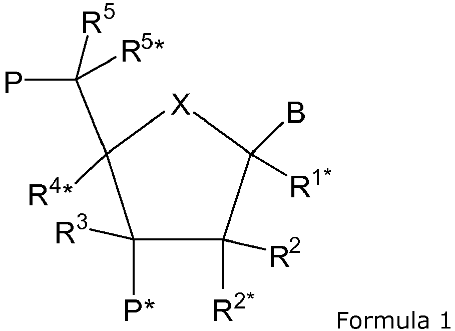
wherein for all chiral centers, asymmetric groups may be found in either R or S orientation;
wherein X is selected from -O-, -S-, -N(R^{N*})-, -C(R⁶R^{6*})-, such as, in some embodiments -O-;
B is selected from hydrogen, optionally substituted C₁₋₄-alkoxy, optionally substituted C₁₋₄-alkyl, optionally substituted C₁₋₄-acyloxy, nucleobases including naturally occurring and nucleobase analogues, DNA intercalators, photochemically active groups, thermochemically active groups, chelating groups, reporter groups, and ligands; preferably, B is a nucleobase or nucleobase analogue;
P designates an internucleotide linkage to an adjacent monomer, or a 5'-terminal group, such internucleotide linkage or 5'-terminal group optionally including the substituent R⁵ or equally applicable the substituent R^{5*};
P* designates an internucleotide linkage to an adjacent monomer, or a 3'-terminal group;
R^{4*} and R^{2*} together designate a bivalent linker group consisting of 1 - 4 groups/atoms selected from -C(R^{a}R^{b})-, -C(R^{a})=C(R^{b})-, -C(R^{a})=N-, -O-, -Si(R^{a})₂-, -S-, -SO₂-, -N(R^{a})-, and >C=Z, wherein Z is selected from -O-, -S-, and -N(R^{a})-, and R^{a} and R^{b} each is independently selected from hydrogen, optionally substituted C₁₋₁₂-alkyl, optionally substituted C₂₋₁₂-alkenyl, optionally substituted C₂₋₁₂-alkynyl, hydroxy, optionally substituted C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)aminO-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted and where two geminal substituents R^{a} and R^{b} together may designate optionally substituted methylene (=CH₂), wherein for all chiral centers, asymmetric groups may be found in either *R* or *S* orientation, and;
each of the substituents R^{1*}, R², R³, R⁵, R^{5*}, R⁶ and R^{6*}, which are present is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted, and where two geminal substituents together may designate oxo, thioxo, imino, or optionally substituted methylene; ; wherein R^{N} is selected from hydrogen and C₁₋₄-alkyl, and where two adjacent (non-geminal) substituents may designate an additional bond resulting in a double bond; and R^{N*}, when present and not involved in a biradical, is selected from hydrogen and C₁₋₄-alkyl; and basic salts and acid addition salts thereof. For all chiral centers, asymmetric groups may be found in either R or S orientation.

In some embodiments, R^{4*} and R^{2*} together designate a biradical consisting of a groups selected from the group consisting of C(R^{a}R^{b})-C(R^{a}R^{b})-, C(R^{a}R^{b})-O-, C(R^{a}R^{b})-NR^{a}-, C(R^{a}R^{b})-S-, and C(R^{a}R^{b})-C(R^{a}R^{b})-O-, wherein each R^{a} and R^{b} may optionally be independently selected. In some embodiments, R^{a} and R^{b} may be, optionally independently selected from the group consisting of hydrogen and C₁₋₆alkyl, such as methyl, such as hydrogen.

In some embodiments, R^{4*} and R^{2*} together designate the biradical -O-CH(CH₂OCH₃)-(2'O-methoxyethyl bicyclic nucleic acid - Seth at al., 2010, J. Org. Chem) - in either the R- or S- configuration.

In some embodiments, R^{4*} and R^{2*} together designate the biradical -O-CH(CH₂CH₃)-(2'O-ethyl bicyclic nucleic acid - Seth at al., 2010, J. Org. Chem). - in either the R- or S-configuration.

In some embodiments, R^{4*} and R^{2*} together designate the biradical -O-CH(CH₃)-. - in either the R- or S- configuration. In some embodiments, R^{4*} and R^{2*} together designate the biradical -O-CH₂-O-CH₂- - (Seth at al., 2010, J. Org. Chem).

In some embodiments, R^{4*} and R^{2*} together designate the biradical -O-NR-CH₃-(Seth at al., 2010, J. Org. Chem).

In some embodiments, the LNA units have a structure selected from the following group:

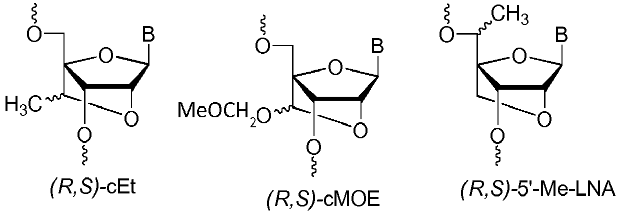

In some embodiments, R^{1*}, R², R³, R⁵, R^{5*} are independently selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl. For all chiral centers, asymmetric groups may be found in either R or S orientation.

In some embodiments, R^{1*}, R², R³, R⁵, R^{5*} are hydrogen.

In some embodiments, R^{1*}, R², R³ are independently selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl. For all chiral centers, asymmetric groups may be found in either R or S orientation.

In some embodiments, R^{1*}, R², R³ are hydrogen.

In some embodiments, R⁵ and R^{5*} are each independently selected from the group consisting of H, -CH₃, -CH₂-CH₃,- CH₂-O-CH₃, and -CH=CH₂. Suitably in some embodiments, either R⁵ or R^{5*} are hydrogen, where as the other group (R⁵ or R^{5*} respectively) is selected from the group consisting of C₁₋₅ alkyl, C₂₋₆ alkenyl, C₂₋₆ alkynyl, substituted C₁₋₆ alkyl, substituted C₂₋₆ alkenyl, substituted C₂₋₆ alkynyl or substituted acyl (-C(=O)-); wherein each substituted group is mono or poly substituted with substituent groups independently selected from halogen, C₁₋₆ alkyl, substituted C₁₋₆ alkyl, C₂₋₆ alkenyl, substituted C₂₋₆ alkenyl, C₂₋₆ alkynyl, substituted C₂₋₆ alkynyl, OJ₁, SJ₁, NJ₁J₂, N₃, COOJ₁, CN, O-C(=O)NJ₁J₂, N(H)C(=NH)NJ,J₂ or N(H)C(=X)N(H)J₂ wherein X is O or S; and each J₁ and J₂ is, independently, H, C₁₋₆ alkyl, substituted C₁₋₆ alkyl, C₂₋₆ alkenyl, substituted C₂₋₆ alkenyl, C₂₋₆ alkynyl, substituted C₂₋₆ alkynyl, C₁₋₆ aminoalkyl, substituted C₁₋₆ aminoalkyl or a protecting group. In some embodiments either R⁵ or R^{5*} is substituted C₁₋₆alkyl. In some embodiments either R⁵ or R^{5*} is substituted methylene wherein preferred substituent groups include one or more groups independently selected from F, NJ₁J₂, N₃, CN, OJ₁, SJ₁, O-C(=O)NJ₁J₂, N(H)C(=NH)NJ, J₂ or N(H)C(O)N(H)J₂. In some embodiments each J₁ and J₂ is, independently H or C₁₋₆ alkyl. In some embodiments either R⁵ or R^{5*} is methyl, ethyl or methoxymethyl. In some embodiments either R⁵ or R^{5*} is methyl. In a further embodiment either R⁵ or R^{5*} is ethylenyl. In some embodiments either R⁵ or R^{5*} is substituted acyl. In some embodiments either R⁵ or R^{5*} is C(=O)NJ₁J₂. For all chiral centers, asymmetric groups may be found in either R or S orientation. Such 5' modified bicyclic nucleotides are disclosed in WO 2007/134181.

In some embodiments B is a nucleobase, including nucleobase analogues and naturally occurring nucleobases, such as a purine or pyrimidine, or a substituted purine or substituted pyrimidine, such as a nucleobase referred to herein, such as a nucleobase selected from the group consisting of adenine, cytosine, thymine, adenine, uracil, and/or a modified or substituted nucleobase, such as 5-thiazolo-uracil, 2-thio-uracil, 5-propynyl-uracil, 2'thio-thymine, 5-methyl cytosine, 5-thiozolo-cytosine, 5-propynyl-cytosine, and 2,6-diaminopurine.

In some embodiments, R^{4*} and R^{2*} together designate a biradical selected from - C(R^{a}R^{b})-O-, -C(R^{a}R^{b})-C(R^{c}R^{d})-O-, -C(R^{a}R^{b})-C(R^{c}R^{d})-C(R^{e}R^{f})-O-, -C(R^{a}R^{b})-O-C(R^{c}R^{d})-,-C(R^{a}R^{b})-O-C(R^{c}R^{d})-O-, -C(R^{a}R^{b})-C(R^{c}R^{d})-, -C(R^{a}R^{b})-C(R^{c}R^{d})-C(R^{e}R^{f})-,-C(R^{a})=C(R^{b})-C(R^{c}R^{d})-, -C(R^{a}R^{b})-N(R^{c})-, -C(R^{a}R^{b})-C(R^{c}R^{d})- N(R^{e})-, -C(R^{a}R^{b})-N(R^{c})-O-, and - C(R^{a}R^{b})-S-, -C(R^{a}R^{b})-C(R^{c}R^{d})-S-, wherein R^{a}, R^{b}, R^{c}, R^{d}, R^{e}, and R^{f} each is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted and where two geminal substituents R^{a} and R^{b} together may designate optionally substituted methylene (=CH₂). For all chiral centers, asymmetric groups may be found in either R or S orientation.

In a further embodiment R^{4*} and R^{2*} together designate a biradical (bivalent group) selected from -CH₂-O-, -CH₂-S-, -CH₂-NH-, -CH₂-N(CH₃)-, -CH₂-CH₂-O-, -CH₂-CH(CH₃)-,-CH₂-CH₂-S-, -CH₂-CH₂-NH-, -CH₂-CH₂-CH₂-, -CH₂-CH₂-CH₂-O-, -CH₂-CH₂-CH(CH₃)-,-CH=CH-CH₂-, -CH₂-O-CH₂-O-, -CH₂-NH-O-, -CH₂-N(CH₃)-O-, -CH₂-O-CH₂-, -CH(CH₃)-O-, and -CH(CH₂-O-CH₃)-O-, and/or, -CH₂-CH₂-, and -CH=CH- For all chiral centers, asymmetric groups may be found in either R or S orientation.

In some embodiments, R^{4*} and R^{2*} together designate the biradical C(R^{a}R^{b})-N(R^{c})-O-, wherein R^{a} and R^{b} are independently selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl, such as hydrogen, and; wherein R^{c} is selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl, such as hydrogen.

In some embodiments, R^{4*} and R^{2*} together designate the biradical C(R^{a}R^{b})-O-C(R^{c}R^{d}) -O-, wherein R^{a}, R^{b}, R^{c}, and R^{d} are independently selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl, such as hydrogen.

In some embodiments, R^{4*} and R^{2*} form the biradical -CH(Z)-O-, wherein Z is selected from the group consisting of C₁₋₆ alkyl, C₂₋₆ alkenyl, C₂₋₆ alkynyl, substituted C₁₋₆ alkyl, substituted C₂₋₆ alkenyl, substituted C₂₋₆ alkynyl, acyl, substituted acyl, substituted amide, thiol or substituted thio; and wherein each of the substituted groups, is, independently, mono or poly substituted with optionally protected substituent groups independently selected from halogen, oxo, hydroxyl, OJ₁, NJ₁J₂, SJ₁, N₃, OC(=X)J₁, OC(=X)NJ₁J₂, NJ³C(=X)NJ₁J₂ and CN, wherein each J₁, J₂ and J₃ is, independently, H or C₁₋₆ alkyl, and X is O, S or NJ₁. In some embodiments Z is C₁₋₆ alkyl or substituted C₁₋₆ alkyl. In some embodiments Z is methyl. In some embodiments Z is substituted C₁₋₆alkyl. In some embodiments said substituent group is C₁₋₆ alkoxy. In some embodiments Z is CH₃OCH₂-. For all chiral centers, asymmetric groups may be found in either R or S orientation. Such bicyclic nucleotides are disclosed in US 7,399,845. In some embodiments, R^{1*}, R², R³, R⁵, R^{5*} are hydrogen. In some some embodiments, R^{1*}, R², R^{3*} are hydrogen, and one or both of R⁵, R^{5*} may be other than hydrogen as referred to above and in WO 2007/134181.

In some embodiments, R^{4*} and R^{2*} together designate a biradical which comprise a substituted amino group in the bridge such as consist or comprise of the biradical -CH₂-N( R^{c})-, wherein R^{c} is C₁₋₁₂ alkyloxy. In some embodiments R^{4*} and R^{2*} together designate a biradical -Cq₃q₄-NOR -, wherein q₃ and q₄ are independently selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl; wherein each substituted group is, independently, mono or poly substituted with substituent groups independently selected from halogen, OJ₁, SJ₁, NJ₁J₂, COOJ₁, CN, O-C(=O)NJ₁J₂, N(H)C(=NH)N J₁J₂ or N(H)C(=X=N(H)J₂ wherein X is O or S; and each of J₁ and J₂ is, independently, H, C₁₋₆ alkyl, C₂₋₆ alkenyl, C₂₋₆ alkynyl, C₁₋₆ aminoalkyl or a protecting group. For all chiral centers, asymmetric groups may be found in either R or S orientation. Such bicyclic nucleotides are disclosed in WO2008/150729. In some embodiments, R^{1*}, R², R³, R⁵, R^{5*} are independently selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl. In some embodiments, R^{1*}, R², R³, R⁵, R^{5*} are hydrogen. In some embodiments, R^{1*}, R², R³ are hydrogen and one or both of R⁵ R^{5*} may be other than hydrogen as referred to above and in WO 2007/134181. In some embodiments R^{4*} and R^{2*} together designate a biradical (bivalent group) C(R^{a}R^{b})-O-, wherein R^{a} and R^{b} are each independently halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxy, substituted C₁-C₁₂ alkoxy, OJ₁ SJ₁, SOJ₁, SO₂J₁, NJ₁J₂, N₃, CN, C(=O)OJ₁, C(=O)NJ₁J₂, C(=O)J₁, O-C(=O)NJ₁J₂, N(H)C(=NH)NJ₁J₂, N(H)C(=O)NJ₁J₂ or N(H)C(=S)NJ₁J₂; or R^{a} and R^{b} together are =C(q3)(q4); q₃ and q₄ are each, independently, H, halogen, C₁-C₁₂alkyl or substituted C₁-C₁₂ alkyl; each substituted group is, independently, mono or poly substituted with substituent groups independently selected from halogen, C₁-C₆ alkyl, substituted C₁-C₆ alkyl, C₂- C₆ alkenyl, substituted C₂-C₆ alkenyl, C₂-C₆ alkynyl, substituted C₂-C₆ alkynyl, OJ₁, SJ₁, NJ₁J₂, N₃, CN, C(=O)OJ₁, C(=O)NJ₁J₂, C(=O)J₁, O-C(=O)NJ₁J₂, N(H)C(=O)NJ₁J₂ or N(H)C(=S)NJ₁J₂. and; each J₁ and J₂ is, independently, H, C1-C₆ alkyl, substituted C1-C₆ alkyl, C₂-C₆ alkenyl, substituted C₂-C₆ alkenyl, C₂-C₆ alkynyl, substituted C₂-C₆ alkynyl, C1-C₆ aminoalkyl, substituted C1-C₆ aminoalkyl or a protecting group. Such compounds are disclosed in WO2009006478A.

In some embodiments, R^{4*} and R^{2*} form the biradical - Q -, wherein Q is C(q₁)(q₂)C(q₃)(q₄), C(q₁)=C(q₃), C[=C(q₁)(q₂)]-C(q₃)(q₄) or C(q₁)(q₂)-C[=C(q₃)(q₄)]; q₁, q₂, q₃, q₄ are each independently. H, halogen, C₁₋₁₂ alkyl, substituted C₁₋₁₂ alkyl, C₂₋₁₂ alkenyl, substituted C₁₋₁₂ alkoxy, OJ₁, SJ₁, SOJ₁, SO₂J₁, NJ₁J₂, N₃, CN, C(=O)OJ₁, C(=O)-NJ₁J₂, C(=O) J₁, -C(=O)NJ₁J₂, N(H)C(=NH)NJ₁J₂, N(H)C(=O)NJ₁J₂ or N(H)C(=S)NJ₁J₂; each J₁ and J₂ is, independently, H, C₁₋₆ alkyl, C₂₋₆ alkenyl, C₂₋₆ alkynyl, C₁₋₆ aminoalkyl or a protecting group; and, optionally wherein when Q is C(q₁)(q₂)(q₃)(q₄) and one of q₃ or q₄ is CH₃ then at least one of the other of q₃ or q₄ or one of q₁ and q₂ is other than H. In some embodiments, R^{1*}, R², R³, R⁵, R^{5*} are hydrogen. For all chiral centers, asymmetric groups may be found in either R or S orientation. Such bicyclic nucleotides are disclosed in WO2008/154401. In some embodiments, R^{1*}, R², R³, R⁵, R^{5*} are independently selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl. In some embodiments, R^{1*}, R², R³, R⁵, R^{5*} are hydrogen. In some embodiments, R^{1*}, R², R³ are hydrogen and one or both of R⁵, R^{5*} may be other than hydrogen as referred to above and in WO 2007/134181 or WO2009/067647 (alpha-L-bicyclic nucleic acids analogs).

In some embodiments the LNA used in the oligonucleotide compounds of the invention preferably has the structure of the general formula II: 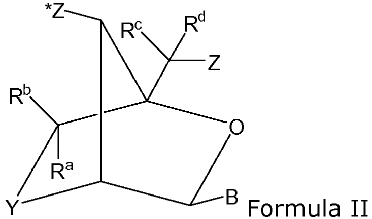 wherein Y is selected from the group consisting of -O-, -CH₂O-, -S-, -NH-, N(R^{e}) and/or-CH₂-; Z and Z* are independently selected among an internucleotide linkage, R^{H}, a terminal group or a protecting group; B constitutes a natural or non-natural nucleotide base moiety (nucleobase), and R^{H} is selected from hydrogen and C₁₋₄-alkyl; R^{a}, R^{b}R^{c}, R^{d} and R^{e} are, optionally independently, selected from the group consisting of hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted and where two geminal substituents R^{a} and R^{b} together may designate optionally substituted methylene (=CH₂); and R^{H} is selected from hydrogen and C₁₋₄-alkyl. In some embodiments R^{a}, R^{b}R^{c}, R^{d} and R^{e} are, optionally independently, selected from the group consisting of hydrogen and C₁₋₆ alkyl, such as methyl. For all chiral centers, asymmetric groups may be found in either R or S orientation, for example, two exemplary stereochemical isomers include the beta-D and alpha-L isoforms, which may be illustrated as follows:

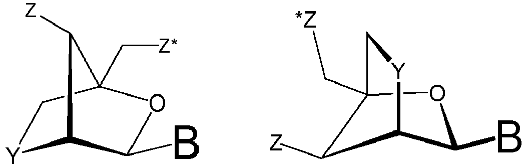

Specific exemplary LNA units are shown below:

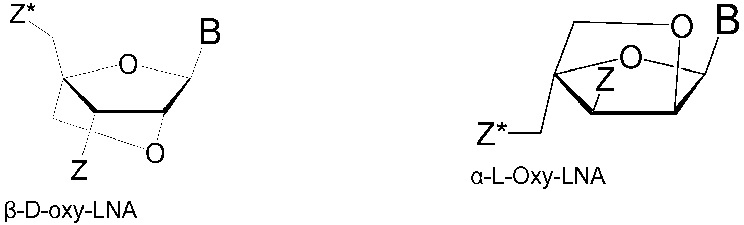

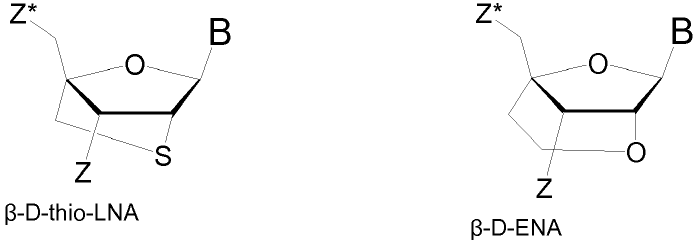

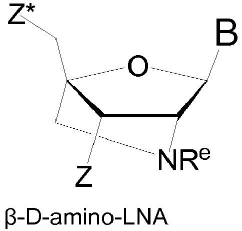

The term "thio-LNA" comprises a locked nucleotide in which Y in the general formula above is selected from S or -CH₂-S-. Thio-LNA can be in both beta-D and alpha-L-configuration.

The term "amino-LNA" comprises a locked nucleotide in which Y in the general formula above is selected from -N(H)-, N(R)-, CH₂-N(H)-, and -CH₂-N(R)- where R is selected from hydrogen and C₁₋₄-alkyl. Amino-LNA can be in both beta-D and alpha-L-configuration.

The term "oxy-LNA" comprises a locked nucleotide in which Y in the general formula above represents -O-. Oxy-LNA can be in both beta-D and alpha-L-configuration.

The term "ENA" comprises a locked nucleotide in which Y in the general formula above is -CH₂-O- (where the oxygen atom of -CH₂-O- is attached to the 2'-position relative to the base B). R^{e} is hydrogen or methyl.
In some exemplary embodiments LNA is selected from beta-D-oxy-LNA, alpha-L-oxy-LNA, beta-D-amino-LNA and beta-D-thio-LNA, in particular beta-D-oxy-LNA.

### Internucleotide Linkages

The monomers of the oligomers described herein are coupled together via linkage groups. Suitably, each monomer is linked to the 3' adjacent monomer via a linkage group.

The person having ordinary skill in the art would understand that, in the context of the present invention, the 5' monomer at the end of an oligomer does not comprise a 5' linkage group, although it may or may not comprise a 5' terminal group.

The terms "linkage group" or "internucleotide linkage" are intended to mean a group capable of covalently coupling together two nucleotides. Specific and preferred examples include phosphate groups and phosphorothioate groups.

The nucleotides of the oligomer of the invention or contiguous nucleotides sequence thereof are coupled together via linkage groups. Suitably each nucleotide is linked to the 3' adjacent nucleotide via a linkage group.

Suitable internucleotide linkages include those listed within WO2007/031091, for example the internucleotide linkages listed on the first paragraph of page 34 of WO2007/031091.

It is, in some embodiments, preferred to modify the internucleotide linkage from its normal phosphodiester to one that is more resistant to nuclease attack, such as phosphorothioate or boranophosphate - these two, being cleavable by RNase H, also allow that route of antisense inhibition in reducing the expression of the target gene.

In some embodiments, such as the embodiments referred to above, where suitable and not specifically indicated, all remaining linkage groups are either phosphodiester or phosphorothioate, or a mixture thereof.

In some embodiments all the internucleotide linkage groups are phosphorothioate.

### Conjugates

In the context the term "conjugate" is intended to indicate a heterogenous molecule formed by the covalent attachment ("conjugation") of the oligomer as described herein to one or more non-nucleotide, or non-polynucleotide moieties. Examples of non-nucleotide or non- polynucleotide moieties include macromolecular agents such as proteins, fatty acid chains, sugar residues, glycoproteins, polymers, or combinations thereof. Typically proteins may be antibodies for a target protein. Typical polymers may be polyethylene glycol.

Therefore, in various embodiments, the oligomer of the invention may comprise both a polynucleotide region which typically consists of a contiguous sequence of nucleotides, and a further non-nucleotide region. When referring to the oligomer of the invention consisting of a contiguous nucleotide sequence, the compound may comprise non-nucleotide components, such as a conjugate component.

In various embodiments of the invention the oligomeric compound is linked to ligands/conjugates, which may be used, e.g. to increase the cellular uptake of oligomeric compounds. WO2007/031091 provides suitable ligands and conjugates.

The invention also provides for a conjugate comprising the compound according to the invention as herein described, and at least one non-nucleotide or non-polynucleotide moiety covalently attached to said compound. Therefore, in various embodiments where the compound of the invention consists of a specified nucleic acid or nucleotide sequence, as herein disclosed, the compound may also comprise at least one non-nucleotide or non-polynucleotide moiety (e.g. not comprising one or more nucleotides or nucleotide analogues) covalently attached to said compound.

Conjugation (to a conjugate moiety) may enhance the activity, cellular distribution or cellular uptake of the oligomer of the invention. Such moieties include, but are not limited to, antibodies, polypeptides, lipid moieties such as a cholesterol moiety, cholic acid, a thioether, e.g. Hexyl-s-tritylthiol, a thiocholesterol, an aliphatic chain, e.g., dodecandiol or undecyl residues, a phospholipids, e.g., di-hexadecyl-rac-glycerol or triethylammonium 1,2-di-o-hexadecyl-rac-glycero-3-h-phosphonate, a polyamine or a polyethylene glycol chain, an adamantane acetic acid, a palmityl moiety, an octadecylamine or hexylamino-carbonyl-oxycholesterol moiety.

The oligomers of the invention may also be conjugated to active drug substances, for example, aspirin, ibuprofen, a sulfa drug, an antidiabetic, an antibacterial or an antibiotic.

In certain embodiments the conjugated moiety is a sterol, such as cholesterol.

In various embodiments, the conjugated moiety comprises or consists of a positively charged polymer, such as a positively charged peptides of, for example from 1 -50, such as 2 - 20 such as 3 - 10 amino acid residues in length, and/or polyalkylene oxide such as polyethylglycol(PEG) or polypropylene glycol - see WO 2008/034123. Suitably the positively charged polymer, such as a polyalkylene oxide may be attached to the oligomer of the invention via a linker such as the releasable inker described in WO 2008/034123.

By way of example, the following conjugate moieties may be used in the conjugates of the invention:

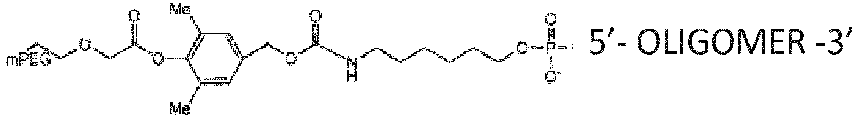

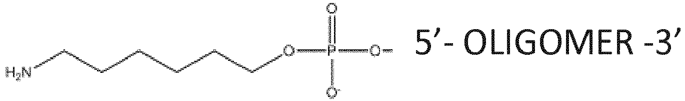

### Activated oligomers

The term "activated oligomer," as used herein, refers to an oligomer of the invention that is covalently linked (i.e., functionalized) to at least one functional moiety that permits covalent linkage of the oligomer to one or more conjugated moieties, i.e., moieties that are not themselves nucleic acids or monomers, to form the conjugates herein described. Typically, a functional moiety will comprise a chemical group that is capable of covalently bonding to the oligomer via, e.g., a 3'-hydroxyl group or the exocyclic NH₂ group of the adenine base, a spacer that is preferably hydrophilic and a terminal group that is capable of binding to a conjugated moiety (e.g., an amino, sulfhydryl or hydroxyl group). In some embodiments, this terminal group is not protected, e.g., is an NH₂ group. In other embodiments, the terminal group is protected, for example, by any suitable protecting group such as those described in "Protective Groups in Organic Synthesis" by Theodora W Greene and Peter G M Wuts, 3rd edition (John Wiley & Sons, 1999). Examples of suitable hydroxyl protecting groups include esters such as acetate ester, aralkyl groups such as benzyl, diphenylmethyl, or triphenylmethyl, and tetrahydropyranyl. Examples of suitable amino protecting groups include benzyl, alpha-methylbenzyl, diphenylmethyl, triphenylmethyl, benzyloxycarbonyl, tert-butoxycarbonyl, and acyl groups such as trichloroacetyl or trifluoroacetyl. In some embodiments, the functional moiety is self-cleaving. In other embodiments, the functional moiety is biodegradable. See e.g., U.S. Patent No. 7,087,229.

In some embodiments, oligomers of the invention are functionalized at the 5' end in order to allow covalent attachment of the conjugated moiety to the 5' end of the oligomer. In other embodiments, oligomers of the invention can be functionalized at the 3' end. In still other embodiments, oligomers of the invention can be functionalized along the backbone or on the heterocyclic base moiety. In yet other embodiments, oligomers of the invention can be functionalized at more than one position independently selected from the 5' end, the 3' end, the backbone and the base.

In some embodiments, activated oligomers of the invention are synthesized by incorporating during the synthesis one or more monomers that is covalently attached to a functional moiety. In other embodiments, activated oligomers of the invention are synthesized with monomers that have not been functionalized, and the oligomer is functionalized upon completion of synthesis. In some embodiments, the oligomers are functionalized with a hindered ester containing an aminoalkyl linker, wherein the alkyl portion has the formula (CH₂)_{w}, wherein w is an integer ranging from 1 to 10, preferably about 6, wherein the alkyl portion of the alkylamino group can be straight chain or branched chain, and wherein the functional group is attached to the oligomer via an ester group (-O-C(O)-(CH₂)_{w}NH).

In other embodiments, the oligomers are functionalized with a hindered ester containing a (CH₂)_{w}-sulfhydryl (SH) linker, wherein w is an integer ranging from 1 to 10, preferably about 6, wherein the alkyl portion of the alkylamino group can be straight chain or branched chain, and wherein the functional group attached to the oligomer via an ester group (-O-C(O)-(CH₂)_{w}SH)

In some embodiments, sulfhydryl-activated oligonucleotides are conjugated with polymer moieties such as polyethylene glycol or peptides (via formation of a disulfide bond).

Activated oligomers containing hindered esters as described above can be synthesized by any method known in the art, and in particular by methods disclosed in PCT Publication No. WO 2008/034122 and the examples therein.

In still other embodiments, the oligomers of the invention are functionalized by introducing sulfhydryl, amino or hydroxyl groups into the oligomer by means of a functionalizing reagent substantially as described in U.S. Patent Nos. 4,962,029 and 4,914,210, i.e., a substantially linear reagent having a phosphoramidite at one end linked through a hydrophilic spacer chain to the opposing end which comprises a protected or unprotected sulfhydryl, amino or hydroxyl group. Such reagents primarily react with hydroxyl groups of the oligomer. In some embodiments, such activated oligomers have a functionalizing reagent coupled to a 5'-hydroxyl group of the oligomer. In other embodiments, the activated oligomers have a functionalizing reagent coupled to a 3'-hydroxyl group. In still other embodiments, the activated oligomers of the invention have a functionalizing reagent coupled to a hydroxyl group on the backbone of the oligomer. In yet further embodiments, the oligomer of the invention is functionalized with more than one of the functionalizing reagents as described in U.S. Patent Nos. 4,962,029 and 4,914,210. Methods of synthesizing such functionalizing reagents and incorporating them into monomers or oligomers are disclosed in U.S. Patent Nos. 4,962,029 and 4,914,210.

In some embodiments, the 5'-terminus of a solid-phase bound oligomer is functionalized with a dienyl phosphoramidite derivative, followed by conjugation of the deprotected oligomer with, e.g., an amino acid or peptide via a Diels-Alder cycloaddition reaction.

In various embodiments, the incorporation of monomers containing 2'-sugar modifications, such as a 2'-carbamate substituted sugar or a 2'-(O-pentyl-N-phthalimido)-deoxyribose sugar into the oligomer facilitates covalent attachment of conjugated moieties to the sugars of the oligomer. In other embodiments, an oligomer with an amino-containing linker at the 2'-position of one or more monomers is prepared using a reagent such as, for example, 5'-dimethoxytrityl-2'-O-(e-phthalimidylaminopentyl)-2'-deoxyadenosine-3'-- N,N-diisopropyl-cyanoethoxy phosphoramidite. See, e.g., Manoharan, et al., Tetrahedron Letters, 1991, 34, 7171.

In still further embodiments, the oligomers of the invention may have amine-containing functional moieties on the nucleobase, including on the N6 purine amino groups, on the exocyclic N2 of guanine, or on the N4 or 5 positions of cytosine. In various embodiments, such functionalization may be achieved by using a commercial reagent that is already functionalized in the oligomer synthesis.

Some functional moieties are commercially available, for example, heterobifunctional and homobifunctional linking moieties are available from the Pierce Co. (Rockford, III.). Other commercially available linking groups are 5'-Amino-Modifier C6 and 3'-Amino-Modifier reagents, both available from Glen Research Corporation (Sterling, Va.). 5'-Amino-Modifier C6 is also available from ABI (Applied Biosystems Inc., Foster City, Calif.) as Aminolink-2, and 3'-Amino-Modifier is also available from Clontech Laboratories Inc. (Palo Alto, Calif.).In some embodimentsin some embodiments

### Compositions

The oligomer of the invention may be used in pharmaceutical formulations and compositions. Suitably, such compositions comprise a pharmaceutically acceptable diluent, carrier, salt or adjuvant. PCT/DK2006/000512 provides suitable and preferred pharmaceutically acceptable diluent, carrier and adjuvants. Suitable dosages, formulations, administration routes, compositions, dosage forms, combinations with other therapeutic agents, pro-drug formulations are also provided in WO2007/031091

### Embodiments

1. A prognostic method for determining the suitability of treatment of a human subject infected with HCV infection, with an inhibitor of microRNA-122, said method comprising the steps of
   i) determining the level of at least one biomarker in the serum sample
   iii) comparing the level of the at least one biomarker with one or more reference samples or reference values,
   to determine whether the subject 1 - 3 to be, or is suitable for, treatment of HCV infection or with an inhibitor of microRNA-122 (i.e. a likely miR-122 inhibitor responder) wherein the at least one biomarker is microRNA-122.
2. The method according to embodiment 1, wherein the inhibitor of micro-RNA-122 is an antisense oligonucleotide which is complementary to microRNA-122 or subsequence thereof, across the length of the oligonucleotide.
3. The method according to embodiment 1 or 2, wherein the subject infected with HCV is treatment naïve and/or asymptomatic and/or has been diagnosed with HCV within the previous 3 years.
4. The method according to any one of embodiments 1 - 3, wherein the at least one biomarker is discriminative with responsiveness to treatment of HCV infection.
5. The method according to embodiment 4, wherein the at least one biomarker is a liver function biomarker.
6. The method according to any one of embodiments 1 - 5, wherein the at least one biomarker further comprises a biomarker selected from the group consisting of: a serum microRNA, GammaGT, AST, and ALT.
7. The method according to embodiments 1 - 6, wherein the microRNA is the mature miR-122.
8. The method according to any one of embodiments 1 - 7, wherein the level of the microRNA biomarker is compared to the level of at least one reference microRNA in the serum sample, such as a microRNA whose levels are invariable in the serum.
9. The method according to embodiment 8, wherein the reference microRNA is selected from one or more of the group consisting of miR-17, miR-18a, miR-345, and miR-16.
10. The method according to any one of embodiments 1 - 7 wherein the at least one biomarker further comprises biomarker which a liver function biomarker, such as a liver enzyme.
11. The method according to embodiment 10 wherein the liver enzyme is selected from the group consisting of ASAT, ALAT, and GGT.
12. The method according to embodiment 10 or 11 wherein the levels of at least 2 liver enzyme biomarkers are determined.
13. The method according to any one of embodiments 1 - 12, wherein the levels of at least 2, 3, or 4 biomarkers are determined.
14. The method according to any one of embodiments 1 - 13 wherein the levels of at least 2 liver enzyme biomarkers are determined as well as the levels of microRNA 122 biomarker.
15. The method according to embodiments 14 wherein the at least 2 liver enzyme biomarkers are selected from the group consisting of ASAT, ALAT, and gammaGT.
16. The method according to embodiment 15 wherein the method comprises the steps of determining the levels of ALAT and/or miR122 and/or GGT in the blood sample, wherein an elevation in ALAT and/or miR-122, ALAT and/or GGT, or ALAT and miR-122 and/or GGT is indicative of a subject who is not suitable for treatment with the miR-122 inhibitor.
17. The method according to embodiment 15 or 16 wherein the method comprises the steps of determining the levels of ALAT and/or miR122 and/or GGT in the blood sample, wherein no elevation in ALAT and/or miR-122, ALAT and/or GGT, or ALAT and miR-122 and/or GGT is indicative of a subject who is suitable for treatment with the miR-122 inhibitor (a [likely] responder.
18. The method according to any one of embodiments 1 - 17, wherein the reference sample or value is obtained from one or more subjects which are not infected with HCV, such as a average value from a comparative non HCV infected healthy population.
19. A prognostic kit for use as an in vitro diagnostic, said kit comprising a quantification assay for human miR-122, and at least one further quantification assay for at least one further biomarker selected from the group consisting of GammaGT, ASAT and ALAT.
20. A method of determining the likely effective dose of a miR-122 inhibitory agent, such as miravirsen, for administration to a subject with HCV infection, said method comprising
   i) determining the level of at least one biomarker in the blood sample
   ii) comparing the level of the at least one biomarker with one or more reference samples or reference values,
   to determine the likely effective dose of the inhibitor of microRNA-122 for administration to the subject in order to alleviate the HCV infection wherein the at least one biomarker is microRNA-122.
21. A detection probe for microRNA-122, for use as a companion diagnostic for a HCV therapeutic, such as a microRNA-122 inhibitor.
22. The detection probe according to embodiment 21 wherein the probe is for use in a prognostic assay for determining the suitability of a subject in need to treatment of (e.g. chronic) HCV for treatment with the HCV therapeutic.
23. The detection probe according to embodiment 21 or 22 wherein the level of liver specific microRNA in serum from is inversely correlated with the suitability of the subject for treatment.

### EXAMPLES

### Transaminase Biomarker Assays:

The biomarker protocols used for measuring ALT was the Advia Chemistry Systems ALT assay (03903166 Rev. B 2007-05); the protocol for measuring ALT was the Advia Chemistry Systems ALT assay (03815151 Rev. B 2007-05), the protocol for measuring GGT was the Advia Chemistry Systems ALT assay (04130756 Rev. B 2007-05).
MicroRNAs were detected using ABI's Taqman assays. Examples of alternative suppliers of microRNAs are provided elsewhere herein, and include for example Exiqon A/S mercury LNA® reagents.

### Example 1: Grading of virological response to treatment with miravirsen

A randomized, double-blind, placebo-controlled, ascending multiple-dose Phase 2a study with 36 treatment-naïve patients with chronic HCV genotype 1 infection were conducted. Patients were enrolled sequentially to one of three cohorts (9 active: 3 placebo per cohort). Miravirsen was given as weekly subcutaneous injections, over 29 days (5 injections in total), at dose 3 mg/kg in cohort 1, 5 mg/kg in cohort 2, and 7 mg/kg in cohort 3.

From all patients included in the study, blood was sampled twice before treatment start: At a screening visit to evaluate suitability for inclusion in the study, and at baseline just before first treatment dose. After treatment start, blood was sampled weekly, until and including week 18 after first treatment dose.

Serum was isolated from each sample, and HCV viral titer was quantified by assessing the HCV RNA levels using qRT-PCR in the samples.

Response to treatment with miravirsen in each patient was graded on a scale from 0 to 4. This grade is assigned based on the log-base-10 maximal reduction in HCV RNA compared to baseline.

### Specifically,

(i) Grade 0: less than 1 log decrease
(ii) Grade 1: more than 1 log and less than 2 log decrease
(iii) Grade 2: more than 2 log and less than 3 log decrease
(iv) Grade 3: more than 3 log and less than 4 log decrease
(v) Grade 4: more than 4 log decrease

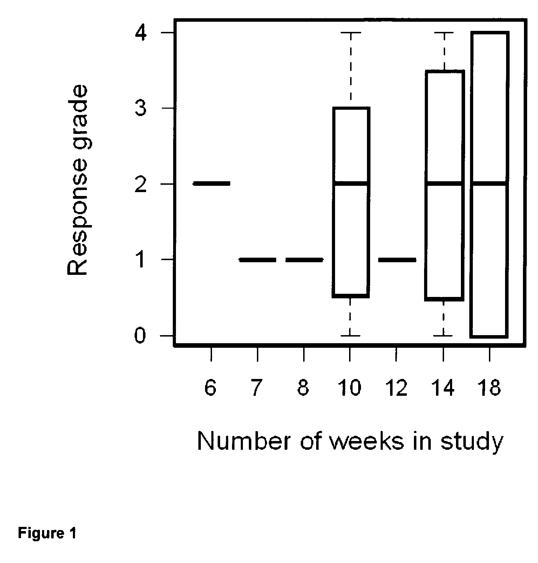

Some patients did not complete the full 18-week study period, but exited the study prematurely for various reasons. In addition, in cohort 3, not all patients have reached week 18 at the time of writing. Since the response grade for a given patient is based on the maximal response observed during the entire study period for that patient, we asked if different study periods would bias the response grade. To this end, we evaluated if there is such a connection between response grade and study period (i.e. number of weeks completed). Fig. 1 shows boxplots of the response grades as a function of the number of weeks completed.
As can be appreciated from Fig. 1, there is no connection between response grade and study period. This demonstrates that the grade is not biased by the shorter-than-18-week study periods for a subset of the patients.

The FDA antiviral products advisory committee defines null responders to treatment of chronic hepatitis C infection as patients with <1 log10 reduction in HCV RNA at week 12 (Sherman et al., 2007 Hepatology Vol. 46 No. 6). Similarly, partial responders are defined as
>1 but <2 log10 reduction in HCV RNA by 12 weeks. For comparison, grade 0 as defined here is therefore a subset of the null responders and grade 1 is a subset of the partial responders.

### Example 2: Response grade dependence on miravirsen dose level

Next, we hypothesized that the virological response to treatment with miravirsen as quantified by the simple five-level grading presented in example 1, could depend on the dose level of the compound.

Thus, for the three dose levels used in the phase 2a study (example 1), we evaluated if there was an association between response grade and dose level.

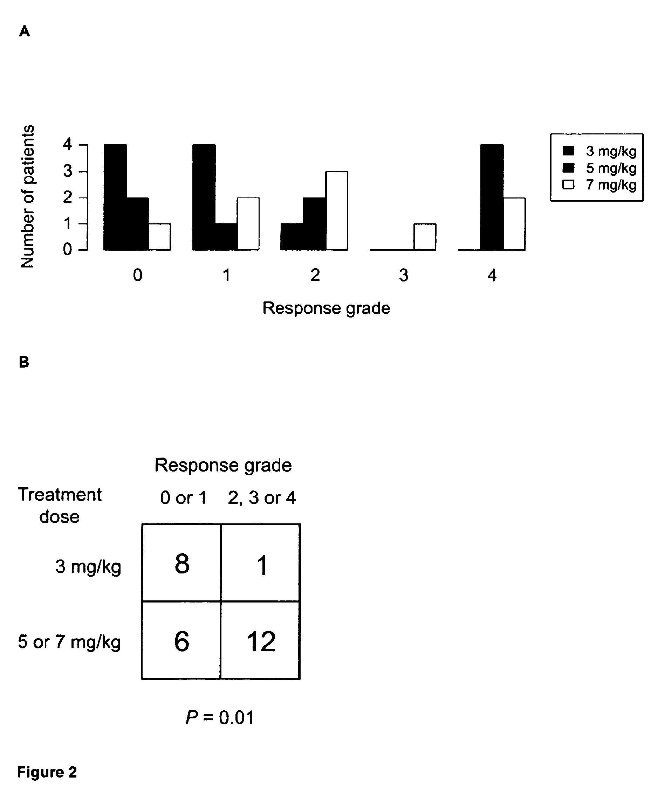

For each response grade, the number of patients at each dose level is shown in Fig. 2A. At the lower grades (0 and 1), the 3 mg/kg dose level is overrepresented. In contrast, at the high grades (3 and 4), the 3 mg/kg dose levels is absent. Comparison of the 5 and 7 mg/kg dose levels shows no clear difference. Statistical analysis of these data using Fisher's Exact test confirms that there is no significant difference in response grades between the 5 and 7 mg/kg dosing levels (*P* = 0.78). If we pool the 5 and 7 mg/kg dose levels and compare this to the 3 mg/kg dosing, the distribution of response grades are borderline significantly different (P = 0.09).

The distribution of response grades as a function of treatment dose is also presented as a contingency table in Fig 2B. Here, response grades are grouped in 0 and 1 versus 2, 3, and 4, and treatment doses in 3 mg/kg versus 5 and 7 mg/kg, to have sufficient patients in each response-treatment dose combination. As seen, most of the high response grades (2, 3 and 4) are reached with the high treatment doses (5 and 7 mg/kg). This association between treatment dose and response grade is statistically significant (P < 0.05 by Fisher's exact test).

Both approaches imply that at least some of the poor responders (grade 0) dosed with the low dose of 3 mg/kg, might have shown a response if dosed with higher concentrations of miravirsen. We thus conclude that the 3 patients dosed at 5 or 7 mg/kg with a grade 0 (see Fig. 2A) most clearly represent patients that respond poorly to therapy with miravirsen.

### Example 3: Measurement of biomarkers in screening and baseline serum samples of HCV patients

All patients included in the phase 2a clinical study described in example 1 had blood drawn at the time of screening (to evaluate inclusion/exclusion in the study) and at the baseline time (prior to first treatment dose). There were between 6 and 34 days between screening and baseline sampling for a given patient, the difference being on average 20 +/- 6 days.

For each sample, 40 different observables were evaluated. These included biomarker measurements as well as function tests and were performed by two clinical chemistry laboratories, except for microRNA quantification that was performed by Asuragen (Austin, Texas, USA), see Table 1.

**Table 1. Observables measured in each sample. For each observable is listed the lower and upper limits for the normal range (for male and females individually when relevant), as well as the unit in which the measurement is reported.**

| **Observable** | **Gender*** | **Lower limit of normal**** | **Upper limit of normal**** | **Unit** |
|---|---|---|---|---|
| ALAT (SGPT) | F and M | 0 | 69 | U/L |
| Albumin | F and M | 34 | 52 | g/L |
| Alkaline Phosphatase | F | 46 | 129 | U/L |
| Alkaline Phosphatase | M | 58 | 141 | U/L |
| APA1 | F and M | 101 | 203 | mg/dL |
| APOB | F and M | 60 | 170 | mg/dL |
| APTT | F and M | 24 | 45 | sec |
| ASAT (SGOT) | F and M | 0 | 52 | U/L |
| B2-microglobulin | F and M | 0 | 300 | ng/mL |
| Basophils | F and M | 0 | 0.2 | 10^9/L |
| Bilirubin (conjugated) | F and M | 0 | 7 | umol/L |
| Bilirubin (total) | F and M | 0 | 30 | umol/L |
| BUN (urea) | F | 0 | 8.1 | mmol/L |
| BUN (urea) | M | 0 | 9.6 | mmol/L |
| Ca (calcium) | F and M | 2.21 | 2.63 | mmol/L |
| Cholesterol | F and M | 0 | 6.5 | mmol/L |
| Cl (chloride) | F and M | 99 | 110 | mmol/L |
| Cockcroft and Gault | F and M | 70 | 160 | mL/min |
| Creatinine | F | 0 | 115 | umol/L |
| Creatinine | M | 0 | 124 | umol/L |
| Eosinophils | F and M | 0 | 0.6 | 10^9/L |
| Factor VII | F and M | 50 | 150 | % |
| GammaGT | F | 0 | 50 | U/L |
| GammaGT | M | 0 | 79 | U/L |
| Globulin | F and M | 20 | 40 | g/L |
| Glucose | F and M | 0 | 6.2 | mmol/L |
| Haemoglobin | F | 7.3 | 9.5 | mmol/L |
| Haemoglobin | M | 8.1 | 11.2 | mmol/L |
| HCV RNA | F and M | 0 | | IU/mL |
| Hematocrit | F | 0.34 | 0.47 | L/L |
| Hematocrit | M | 0.38 | 0.52 | L/L |
| Inorganic phosphate | F and M | 0.8 | 1.5 | mmol/L |
| K (potassium) | F and M | 3.6 | 5.2 | mmol/L |
| LDH | F and M | 0 | 275 | U/L |
| Leucocyte count | F and M | 3.8 | 11 | 10^9/L |
| Lymfocytes | F and M | 1 | 4.8 | 10^9/L |
| Monocytes | F and M | 0 | 0.9 | 10^9/L |
| Na (sodium) | F and M | 137 | 147 | mmol/L |
| Neutrophils | F and M | 1.4 | 8.2 | 10^9/L |
| Platelet count | F and M | 150 | 400 | 10^9/L |
| Protein (total) | F and M | 63 | 85 | g/L |
| PT | F and M | 10 | 15 | sec |
| PT (INR) | F and M | 0.85 | 3.5 | |
| PH in urine | F and M | 5 | 8 | |
| Urine specific gravity | F and M | 1.003 | 1.035 | kg/L |
| microRNA-122 | F and M | -2.4 | 1.0 | cycles |

| | | | | |
|---|---|---|---|---|
| * F: female, M: male ** defined as average +/- 2 standard deviations. When differences between European and US clinical chemistry laboratory standard ranges are reported, the maximal range was chosen. For miR-122, the standard ranges were calculated as described in the example. | | | | |

The clinical chemistry laboratory measurements were performed as described elsewhere.

The microRNA measurements were performed in baseline serum samples only. In brief, RNA was extracted from each serum sample using the extraction method established by Asuragen's Pharmacogenomics Services Group. In each RNA sample, the levels of miR-122, miR-17, and miR-18a were measured by qRT-PCR. These measurements were performed as a two-step RT-PCR procedure using TaqMan Small RNA Assays (Applied Biosystems; Foster City, CA, USA). The first step involved cDNA synthesis from total RNA using a target specific stem-loop reverse-transcriptase primer, resulting in generation of a mature miRNA/RT-primer chimeric amplicon. In the second step, this amplicon was amplified in a standard TaqMan real-time PCR assay using amplicon specific forward and reverse primers. In addition to the primers, a fluorescently labeled and amplicon specific TaqMan probe was used to monitor the increase in amount of amplicon as the PCR progressed. The cycle number at which the fluorescence signal from the TaqMan probe exceeds a threshold value above noise, defined as the Ct value, is used as a measure of the original concentration of cDNA in the sample (the lower the Ct value, the fewer cycles have been necessary to amplify the signal above noise, and the more starting cDNA material there must have been). In an independent study, we have identified miR-17 and miR-18a as stably expressed between HCV infected patients and healthy control subjects. We therefore averaged the Ct values for miR-17 and miR-18a (mean Ct of miR-17 and miR-18a), and subtracted the Ct value for miR-122 from this mean value. This establishes a dCt value for miR-122 that is comparable across all the serum samples, dCt(miR-122) = (Ct(miR-17)+Ct(miR-18a))/2 - Ct(miR-122). Specifically, the larger the dCt value, the larger the expression of miR-122 in the sample.

To evaluate robustness over time, for each observable in each sample, we evaluated the relation between screening and baseline measurements. Specifically, for each observable, we calculated the correlation between screening and baseline measurements across patients. The results are shown in Table 2, first two columns. As seen, 20 of the observables show a strong and significant correlation (*r* ≥ 0.7, *P* < 0.0001) between the measurements at screening and baseline, and 6 observables show weak or no correlation (*r* < 0.4, *P* > 0.01).

**Table 2. Comparison of measurements at screening and baseline. For those 33 observables that were measured in all patients at both screening and baseline times, two comparisons are listed. In columns 1 and 2 are shown for each observable the correlation coefficient and its associated significance when comparing screening and baseline values across patients (Spearman's rank correlation test). In column 3 is shown the significance when evaluating the difference in averages at screening and baseline (Wilcoxon signed-rank test).**

| In addition to the correlation we also evaluated general shifts in the measured values at baseline and screening times. The significance of such a shift in values is listed in column 3 in Table 2. Only haematocrit, haemoglobin, and calcium (borderline significance) are judged as displaying significant overall shifts in value (P < 0.005). | | | |
|---|---|---|---|
| **Observable** | ***r*** | ***P* (correlation)** | ***P* (wilcoxon)** |
| Cockcroft and Gault | 0.93 | 1.20E-14 | 0.95 |
| Globulin | 0.91 | 3.00E-14 | 0.13 |
| Cholesterol | 0.9 | 1.00E-13 | 0.017 |
| Alkaline Phosphatase | 0.87 | 8.50E-12 | 0.44 |
| HCV RNA | 0.87 | 8.10E-12 | 0.21 |
| GammaGT | 0.84 | 1.70E-10 | 0.76 |
| Platelet count | 0.84 | 4.20E-10 | 0.41 |
| ALAT (SGPT) | 0.83 | 2.50E-10 | 0.8 |
| ASAT (SGOT) | 0.81 | 1.60E-09 | 0.56 |
| Haemoglobin | 0.81 | 3.20E-09 | 0.0018 |
| Protein (total) | 0.78 | 2.60E-08 | 0.032 |
| Creatinine | 0.77 | 3.60E-08 | 0.27 |
| Monocytes | 0.75 | 1.70E-07 | 0.32 |
| Hematocrit | 0.75 | 2.80E-07 | 5.70E-05 |
| Bilirubin (conjugated) | 0.74 | 2.90E-07 | 0.54 |
| LDH | 0.71 | 1.50E-06 | 0.3 |
| PT | 0.7 | 2.10E-06 | 0.31 |
| Leucocyte count | 0.7 | 2.40E-06 | 0.32 |
| Bilirubin (total) | 0.7 | 1.80E-06 | 1 |
| Lymfocytes | 0.7 | 2.80E-06 | 0.25 |
| APTT | 0.62 | 6.30E-05 | 0.39 |
| Neutrophils | 0.62 | 0.00017 | 0.76 |
| Albumin | 0.62 | 5.50E-05 | 0.021 |
| BUN (urea) | 0.62 | 6.30E-05 | 0.043 |
| Eosinophils | 0.6 | 0.00013 | 0.52 |
| Ca (calcium) | 0.52 | 0.001 | 0.0051 |
| Cl (chloride) | 0.4 | 0.016 | 0.15 |
| K (potassium) | 0.4 | 0.016 | 0.027 |
| Urine specific gravity | 0.32 | 0.061 | 0.3 |
| pH in urine | 0.24 | 0.15 | 0.17 |
| Basophils | 0.23 | 0.18 | 0.85 |
| Glucose | 0.15 | 0.37 | 0.34 |

### Example 4: Identifying associations between baseline levels of observables and miravirsen response grade

We identified associations between baseline levels of observables (see example 3) and response to treatment with miravirsen (see example 1) using two approaches: Either by a significant difference in average measurement levels between poor responders (grade 0) and the rest (grades 1-4), as evaluated by Wilcoxon rank sum test, or by a significant correlation between measurement levels and response grade, as evaluated by Spearman's rank correlation test.

We include patients from all three cohorts in this analysis even though some of the grade 0 responders in the low dose group might have shown a higher grade response if they had been administered a higher dose (see example 2).

Out of the 40 observables for which we have baseline data, the four that most significantly associated with response grade as evaluated by rank sum- and/or rank correlation test, and also showed a high concordance between screening and baseline values (*r* > 0.8, see example 3), were miR-122, GGT, ALAT, and ASAT, see Fig. 3 and Table 3.

**Table 3. Significant associations between observables and response grades. Significance from Wilcoxon rank sum test (relating to barplots in Fig.3) and results from Spearman's rank correlation test (relating to scatterplots in Fig.3). Only the top-5 is shown.**

| **Observable** | ***P* (wilcox)** | ***r*** | ***P* (correlation)** |
|---|---|---|---|
| miR-122 | 0.005 | -0.48 | 0.01 |
| PT | 0.14 | 0.44 | 0.02 |
| GGT | 0.31 | -0.39 | 0.05 |
| ALAT | 0.05 | -0.35 | 0.07 |
| ASAT | 0.13 | -0.30 | 0.12 |

These four observables have all beeen reported as serum biomarkers of liver function or liver disease/damage (for ALAT, ASAT, and GGT this is well known, for miR-122, see Bihrer et al., 2011, Am. J. Gastroenterol, Vol. 106, No. 6).

Also, PT was identified as having a significant correlation with response (table 3).

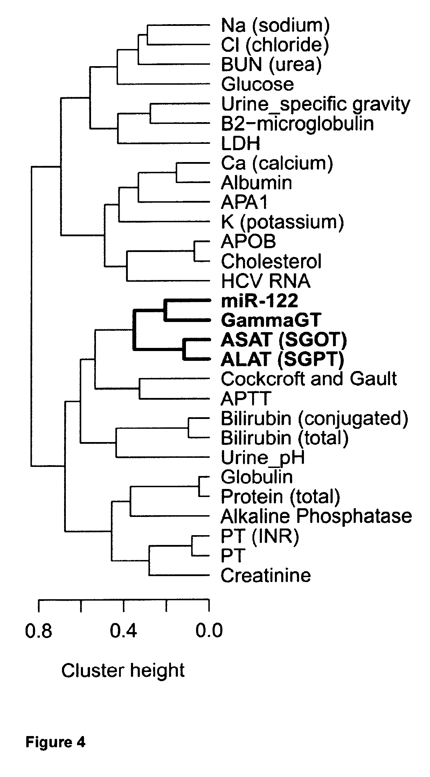

In addition, for all observables measured, we calculated pairwise correlations. From this we cluster observables based on the degree of positive correlation between them, see Fig. 4 below. As seen, miR-122, GGT (GammaGT), ALAT, and ASAT cluster together because of loose pairwise correlations (average pairwise correlations r= 0.46).

### Example 5: Constructing a prognostic classifier

In example 4 we (a) identified significant associations between parameters of liver function, specifically the levels of each of the observables ALAT, ASAT, GGT, and miR-122, and the miravirsen treatment response grade. In addition, we (b) showed that these four biomarkers correlated with each other, but only loosely so. Finally, in example 3 we (c) showed that for ALAT, ASAT, GGT, where also screening time measurements were available, in most patients the measurement at screening and baseline were very similar. These three properties, (a) significance, (b) independence, and (c) robustness, motivated us to explore the utility of one or more of these observables to establish an accurate prediction of the response grade.

Our predictive method is constructed by means of a classification algorithm known as a decision tree (Duda, Hart, and Stork, Pattern Classification, Wiley-Interscience; 2nd edition, 2000), specifically a binary decision tree.

A binary decision tree can be understood as a sequence of questions, or decisions, in which the next question asked depends on the answer, either yes or no, to the current question. Such a sequence of decisions can be presented in a tree-like structure consisting of nodes (the questions) connected by branches (the answers) that lead to other nodes. By convention, the first node (first question) is displayed at the top, so that the tree is upside-down.

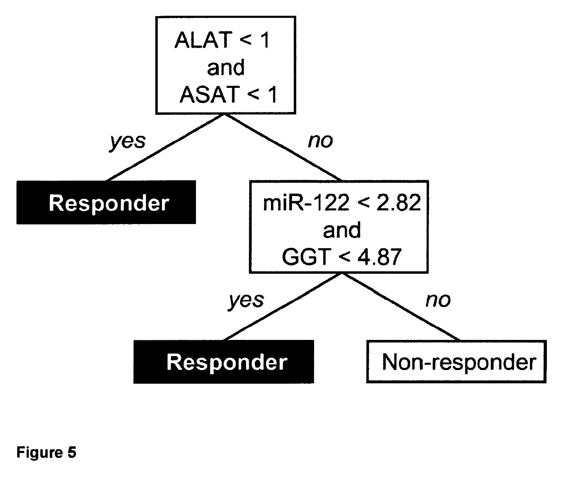

In our case, for a given sample in which ALAT, ASAT, GGT, and miR-122 have been measured, the classification by binary decision tree then proceeds as a sequence of questions, where each question evaluates whether one or more of these observables is equal to or lies above, a certain cut-off value. This scheme is analogous to a pathologist's workup process, wherein a sample is assigned to increasingly finer subgroups through a series of differential diagnosis tests. A specific example is shown in Fig. 5

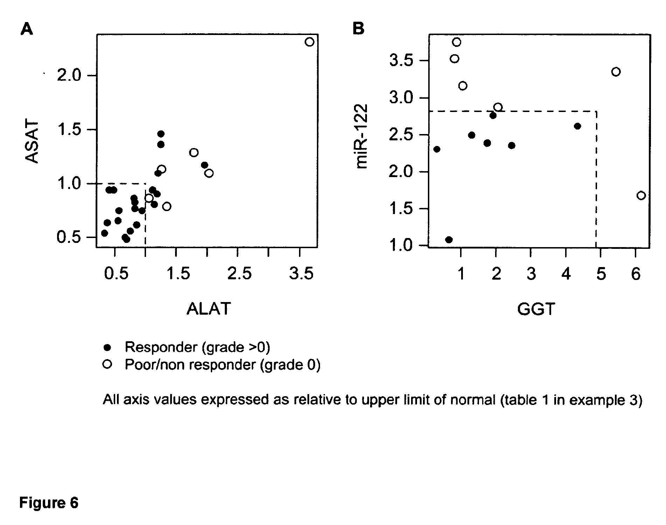

The rationale for the tree structure in Fig. 5 is presented in Fig. 6. In Fig 6A, the ALAT and ASAT measurements (expressed relative to the upper limit of normal, see example 3) for all patients treated with miravirsen are plotted against each other. All patients with ALAT < 1 and ASAT < 1 (dashed lines in Fig. 6A), responded to treatment (response grade >0). This observation is captured by the first question (root node) in Fig. 5. In Fig 6B are shown only those 13 patients that did not have ALAT < 1 and ASAT < 1. For these patients, the miR-122 and GGT measurements are plotted against each other. For miR-122 < 2.82 and GGT < 4.87 (dashed lines in Fig. 6B) the remaining patients are completely separated into those that respond, and those that do not respond, to treatment with miravirsen. This observation is captured by the second question in Fig. 5.

This classification model implies that patients responding poorly, or not at all, to treatment with miravirsen, have a challenged liver function or liver damage: they have elevated levels of ALAT and/or ASAT, compared to upper limit of normal, and highly elevated levels of miR-122 and/or GGT, compared to upper limit of normal.

### Example 6: Training and testing the classifier

Turning now to the matter of using training data to create or "grow" a decision tree. Any decision tree will progressively split the samples into smaller and smaller subsets. Ideally, each subset should eventually only contain samples assigned the same label (for example grade 0). Also, preferably only a single or a few subsets should contain samples with any given label. Finally, we preferred a simple, compact tree, in reference to the general observation that the simplest model that explains the data is generally the one to prefer.

Several different mathematical measures that optimize the single-label "purity" in subgroups after a split have been proposed (Duda, Hart, and Stork, Pattern Classification, Wiley-Interscience; 2nd edition, 2000). One example is the variance impurity measure *i*(*N*) = *P₁P₂*, where *Pⱼ* is the fraction of samples at node *N* that have label *j*. Using this measure, the observable at a given cutoff that maximizes the drop in impurity after the split, Δ*i*(*N*), can found by exhaustive searching in combination with gradient descent algorithms. Here, the drop in impurity Δ*i*(*N*) is defined as Δ*i*(*N*) = *i*(*N*) *- P_{L}i*(*N_{L}*) *-* (1-*P_{L}*)*i*(*N_{R}*), where *N_{L}* and *N_{R}* are the left and right descendant nodes, *i*(*M_{L}*) and *i*(*N_{R}*) are their impurities, and *P_{L}* is the fraction of patterns at node *N* that will go to *N_{L}* when a given observable and cutoff is used.

Such measures work best when the training data is representative of all the biomarker measurements that the classifier will encounter when tested. In practice this means that the number of patients included in the training data needs to be high enough that it is possible to remove a fraction of patients in the training data, say 5-10%, without significantly affecting the structure and cutoffs in the tree. Such a trained tree is then said to be robust.

An unbiased estimate of the predictive performance (accuracy, specificity, sensitivity, etc.) of such a robustly trained classifier can be established using an independent new set of patients; the test data. Most importantly, the test data must not have been used in any way to train the classifier.

The biomarkers included in a robustly trained tree by the methods described here, as well as the cutoff values identified, can be accepted as generally predictive when a high degree of accuracy is achieved upon applying the classifier to an independent test set of patients.

## Claims

1. A prognostic method for determining the suitability of treatment of a human subject infected with HCV infection, with an inhibitor of microRNA-122, said method comprising the steps of:
i) determining the level of at least one biomarker in a serum sample obtained from said human subject;
ii) comparing the level of the at least one biomarker with one or more reference samples or reference values;
to determine whether the subject is likely to be, or is suitable for, treatment of HCV infection with an inhibitor of microRNA-122 (i.e. a likely miR-122 inhibitor responder); wherein the at least one biomarker is microRNA-122.

2. The method according to claim 1, wherein the inhibitor of micro-RNA-122 is an antisense oligonucleotide which is complementary to microRNA-122 or subsequence thereof, across the length of the oligonucleotide.

3. The method according to claim 2, wherein the antisense oligonucleotide is a LNA oligonucleotide.

4. The method according to claim 2, wherein the antisense oligonucleotide is miravirsen.

5. The method according to any one of claims 1 - 4, wherein the subject infected with HCV is treatment naïve and/or asymptomatic and/or has been diagnosed with HCV within the previous 3 years.

6. The method according to any one of claims 1 - 5, wherein the step i) of the prognostic method further comprises the step of determining the level of at least one further biomarker in the a serum sample obtained from said human subject, and comparing the level of the at least one further biomarker with one or more reference samples or reference values, wherein the at least one further biomarker is a liver enzyme biomarker selected from the group consisting of GammaGT, ASAT, and ALAT.

7. The method according to claim 6, wherein the at least one further biomarker is ASAT.

8. The method according to claim 7, wherein the at least one further biomarker is ALAT.

9. The method according to claim 8, wherein the at least one further biomarker is GammaGT.

10. The method according to any one of claims 6 - 9, wherein the levels of at least 2 of the liver enzyme biomarkers are determined.

11. The in vitro use of a detection probe for microRNA-122 as a prognostic assay for determining the suitability of a subject in need of treatment for (e.g. chronic) HCV, for treatment with a HCV therapeutic, such as a microRNA-122 inhibitor.

12. A kit comprising the detection probe according to claim 11, for use as an *in vitro* diagnostic for in determining the suitability of a subject in need of treatment for (e.g. chronic) HCV treatment with a HCV therapeutic, said kit comprising a quantification assay for human miR-122, and at least one further quantification assay for at least one further biomarker selected from the group consisting of GammaGT, ASAT, and ALAT.

13. A method of determining the likely effective dose of a miR-122 inhibitory agent, for administration to a subject with HCV infection, said method comprising the steps of
i) determining the level of at least one biomarker in the blood sample obtained from the human subject infected with HCV;
ii) comparing the level of the at least one biomarker with one or more reference samples or reference values,
to determine the likely effective dose of the inhibitor of microRNA-122 for administration to the subject in order to alleviate the HCV infection; wherein the at least one biomarker is microRNA-122.

14. The method according to claim 13, wherein the miR-122 inhibitory agent is a LNA oligonucleotide.

15. The method according to claim 13, wherein the miR-122 inhibitory agent is miravirsen.

## Patentansprüche

1. Prognoseverfahren zur Bestimmung der Eignung einer Behandlung eines humanen Patienten mit einer HCV-Infektion mit einem Inhibitor von MikroRNA-122, wobei das Verfahren folgende Schritte umfasst:
i) Bestimmen der Konzentration mindestens eines Biomarkers in einer Serumprobe, die dem humanen Patienten entnommen wurde;
ii) Vergleichen der Konzentration des mindestens einen Biomarkers mit einer oder mehreren Vergleichsproben oder Vergleichswerten;
zur Bestimmung, ob der Patient möglicherweise oder sicher geeignet für die Behandlung der HCV-Infektion mit einem Inhibitor von MikroRNA-122 (d. h. ein wahrscheinlicher miR-122-Inhibitor-Responder) ist; wobei der mindestens eine Biomarker MikroRNA-122 ist.

2. Verfahren nach Anspruch 1, wobei der Inhibitor von MikroRNA-122 ein Antisense-Oligonukleotid ist, das über die Länge des Oligonukleotids komplementär zu MikroRNA-122 oder einer Untersequenz davon ist.

3. Verfahren nach Anspruch 2, wobei das Antisense-Oligonukleotid ein LNA-Oligonukleotid ist.

4. Verfahren nach Anspruch 2, wobei das Antisense-Oligonukleotid Miraversen ist.

5. Verfahren nach einem der Ansprüche 1-4, wobei der mit HCV infizierte Patient behandelungsnaiv ist und/oder asymptomatisch ist und/oder die HCV-Diagnose innerhalb der letzten drei Jahre erhalten hat.

6. Verfahren nach einem der Ansprüche 1-5, wobei Schritt i) des Prognoseverfahrens weiterhin den Schritt des Bestimmens der Konzentration mindestens eines weiteren Biomarkers in der dem humanen Patienten entnommenen Serumprobe und des Vergleichens der Konzentrationen des mindestens einen weiteren Biomarker mit einer oder mehreren Vergleichsproben oder Vergleichswerten umfasst, wobei der mindestens eine weitere Biomarker ein Leberenzym-Biomarker ist, ausgewählt aus der Gruppe, bestehend aus GammaGT, ASAT und ALAT.

7. Verfahren nach Anspruch 6, wobei der mindestens eine weitere Biomarker ASAT ist.

8. Verfahren nach Anspruch 7, wobei der mindestens eine weitere Biomarker ALAT ist.

9. Verfahren nach Anspruch 8, wobei der mindestens eine weitere Biomarker GammaGT ist.

10. Verfahren nach einem der Ansprüche 6-9, wobei die Konzentration von mindestens zwei der Leberenzym-Biomarker bestimmt werden.

11. In-vitro-Verwendung einer Nachweissonde für MikroRNA-122 als Prognosetest zur Bestimmung der Eignung eines Patienten, der eine Behandlung gegen (beispielsweise chronisches) HCV benötigt, zur Behandlung mit einem HCV-Therapeutikum, wie einem MikroRNA-122-Inhibitor.

12. Kit, umfassend die Nachweissonde nach Anspruch 11, zur Verwendung als ein In-vitro-Diagnostikum zur Bestimmung der Eignung eines Patienten, der eine Behandlung gegen (beispielsweise chronisches) HCV benötigt, für ein HCV-Therapeutikum, wobei der Kit einen Quantifizierungstest für humane miR-122 umfasst und mindestens einen weiteren Quantifizierungstest für mindestens einen weiteren Biomarker, ausgewählt aus der Gruppe, bestehend aus GammaGT, ASAT und ALAT.

13. Verfahren zur Bestimmung wahrscheinlich wirksamen Dosis eines miR-122 hemmenden Mittels zur Verabreichung an einen Patienten mit einer HCV-Infektion, wobei das Verfahren folgende Schritte umfasst:
i) Bestimmen der Konzentration mindestens eines Biomarkers in einer Blutprobe, die dem humanen mit HCV infizierten Patienten entnommen wurde;
ii) Vergleichen der Konzentration des mindestens einen Biomarkers mit einer oder mehreren Vergleichsproben oder Vergleichswerten;
Bestimmen der wahrscheinlich wirksamen Dosis des MikroRNA-122-Inhibitors zur Verabreichung an den Patienten, um die HCV-Infektion zu lindern; wobei der mindestens eine Biomarker MikroRNA-122 ist.

14. Verfahren nach Anspruch 13, wobei das miR-122 hemmende Mittel ein LNA-Oligonukleotid ist.

15. Verfahren nach Anspruch 13, wobei das miR-122 hemmende Mittel Miraversen ist.

## Revendications

1. Méthode pronostique pour déterminer l'aptitude d'un sujet humain infecté par une infection à VHC au traitement avec un inhibiteur du microARN-122, ladite méthode comprenant les étapes suivantes :
i) détermination du niveau d'au moins un biomarqueur dans un échantillon de sérum prélevé chez ledit sujet humain ;
ii) comparaison du niveau de l'au moins un biomarqueur avec un ou plusieurs échantillons de référence ou valeurs de référence,
afin de déterminer si le sujet est susceptible d'être, ou est apte à être, traité pour une infection à VHC avec un inhibiteur du microARN-122 (c'est-à-dire susceptible d'être répondeur à l'inhibiteur du miR-122) ;
dans laquelle l'au moins un biomarqueur est le microARN-122.

2. La méthode selon la revendication 1, dans laquelle l'inhibiteur du microARN-122 est un oligonucléotide antisens qui est complémentaire du microARN-122 ou d'une sous-séquence de celui-ci, sur toute la longueur de l'oligonucléotide.

3. La méthode selon la revendication 2, dans laquelle l'oligonucléotide antisens est un oligonucléotide de LNA.

4. La méthode selon la revendication 2, dans laquelle l'oligonucléotide antisens est du miraversen.

5. La méthode selon l'une quelconque des revendications 1 à 4, dans laquelle le sujet infecté par le VHC n'a jamais reçu de traitement et/ou est asymptomatique et/ou a été diagnostiqué comme étant atteint du VHC au cours des trois années précédentes.

6. La méthode selon l'une quelconque des revendications 1 à 5, dans laquelle l'étape i) de la méthode pronostique comprend également l'étape de détermination du niveau d'au moins un autre biomarqueur dans l'échantillon de sérum prélevé chez ledit sujet humain, et la comparaison du niveau de l'au moins un autre biomarqueur avec un ou plusieurs échantillons de référence ou valeurs de référence, dans laquelle l'au moins un autre biomarqueur est un biomarqueur des enzymes hépatiques choisi dans le groupe consistant en gammaGT, ASAT et ALAT.

7. La méthode selon la revendication 6, dans laquelle l'au moins un autre biomarqueur est l'ASAT.

8. La méthode selon la revendication 7, dans laquelle l'au moins un autre biomarqueur est l'ALAT.

9. La méthode selon la revendication 8, dans laquelle l'au moins un autre biomarqueur est la gammaGT.

10. La méthode selon l'une quelconque des revendications 6 à 9, dans laquelle les niveaux d'au moins 2 des biomarqueurs des enzymes hépatiques sont déterminés.

11. L'utilisation *in vitro* d'une sonde de détection du microARN-122 comme test pronostique pour déterminer l'aptitude d'un sujet nécessitant un traitement contre le VHC (par exemple chronique) au traitement avec un agent thérapeutique contre le VHC, tel qu'un inhibiteur du microARN-122.

12. Kit comprenant la sonde de détection selon la revendication 11, pour utilisation comme diagnostic *in vitro* pour déterminer l'aptitude d'un sujet nécessitant un traitement contre le VHC (par exemple chronique) au traitement avec un agent thérapeutique contre le VHC, ledit kit comprenant un test de quantification du miR-122 humain, et au moins un autre test de quantification d'au moins un autre biomarqueur choisi dans le groupe consistant en gammaGT, ASAT et ALAT.

13. Méthode de détermination de la dose efficace probable d'un agent inhibiteur du miR-122 pour administration à un sujet infecté par le VHC, ladite méthode comprenant les étapes suivantes :
i) détermination du niveau d'au moins un biomarqueur dans l'échantillon de sang prélevé chez le sujet humain infecté par le VHC ;
ii) comparaison du niveau de l'au moins un biomarqueur avec un ou plusieurs échantillons de référence ou valeurs de référence,
afin de déterminer la dose efficace probable de l'inhibiteur du microARN-122 à administrer au sujet pour atténuer l'infection par le VHC, dans laquelle l'au moins un biomarqueur est le microARN-122.

14. La méthode selon la revendication 13, dans laquelle l'agent inhibiteur du miR-122 est un oligonucléotide de LNA.

15. La méthode selon la revendication 13, dans laquelle l'agent inhibiteur du miR-122 est le miraversen.
